(19) Europäisches Patentamt
European Patent Office
Office européen des brevets

(11) **EP 2 845 906 B1**

(12) **FASCICULE DE BREVET EUROPEEN**

(45) Date de publication et mention
de la délivrance du brevet:
**17.10.2018 Bulletin 2018/42**

(51) Int Cl.:
*C12Q 1/02* (2006.01)          *A01C 21/00* (2006.01)
*C05F 11/08* (2006.01)

(21) Numéro de dépôt: **14290257.6**

(22) Date de dépôt: **27.08.2014**

(54) **Procédé d'évaluation de l'efficience carbonée des bactéries du sol**

Bewertungsverfahren der Kohlendioxideffizienz von Bodenbakterien

Method for assessing the carbon efficiency of soil bacteria

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité: **06.09.2013 FR 1302069**

(43) Date de publication de la demande:
**11.03.2015 Bulletin 2015/11**

(73) Titulaire: **Polyor SARL
54000 Nancy (FR)**

(72) Inventeur: **CLAUDE, Pierre-Philippe
54000 Nancy (FR)**

(56) Documents cités:
EP-A1- 2 223 586     EP-A1- 2 227 936
EP-A1- 2 409 561

• S. MANZONI ET AL: "The Global Stoichiometry of Litter Nitrogen Mineralization", SCIENCE, vol. 321, no. 5889, 1 août 2008 (2008-08-01), pages 684-686, XP055123967, ISSN: 0036-8075, DOI: 10.1126/science.1159792
• STEFANO MANZONI ET AL: "Environmental and stoichiometric controls on microbial carbon-use efficiency in soils", NEW PHYTOLOGIST, vol. 196, no. 1, 11 juillet 2012 (2012-07-11), pages 79-91, XP055124488, ISSN: 0028-646X, DOI: 10.1111/j.1469-8137.2012.04225.x
• ROBERT L. SINSABAUGH ET AL: "Carbon use efficiency of microbial communities: stoichiometry, methodology and modelling", ECOLOGY LETTERS, vol. 16, no. 7, 30 avril 2013 (2013-04-30) , pages 930-939, XP055124489, ISSN: 1461-023X, DOI: 10.1111/ele.12113
• GANG CHEN ET AL: "Soil microbial activities and carbon and nitrogen fixation", RESEARCH IN MICROBIOLOGY, vol. 154, no. 6, 1 juillet 2003 (2003-07-01), pages 393-398, XP055124485, ISSN: 0923-2508, DOI: 10.1016/S0923-2508(03)00082-2

**Description**

**DOMAINE TECHNIQUE DE L'INVENTION**

Analyse, diagnostique et préconisation d'échantillons agro-environnementaux, et plus particulièrement de sols arables. Il est aussi question de biofertilisation azotobactérienne, notamment en présence de résidus de culture cellulosiques et/ou pailleux au sol.

**ÉTAT DE LA TECHNIQUE**

EFFICIENCE CARBONÉE (EC) DE LA MICROFLORE DU SOL

L'utilisation de microorganismes pouvant valoriser les biomasses souterraines d'origines végétales, notamment les bactéries diazotrophes et/ou *Azotobacteraceae* appliquées sur résidus de culture cellulosiques au sol (Claude et Fillion 2004), est en développement. En principe, l'efficacité relative à un témoin non inoculé de la sorte à l'aide de tels microorganismes dépendra ;

> du degré d'endogénécité (*cf.* FR 01 01542) des bactéries ainsi réintroduites du fait qu'elles ne peuvent bénéficiers pour la plupart de symbioses spécifiques les mettant à l'abri de l'environnement physico-chimique, foncièrement hostile, du sol ;
> de leur niveau d'activité diazotrophe du fait qu'elles doivent le plus souvent apporter par réduction enzymatique (*Nif* ; nitrogénase) du $N_2$ de l'azote à la résidusphère ;
> de leur capacité à s'imposer entant que populations au sein de la microflore du sol afin de ne pas être complètement supplantées, notamment par les flores fongiques les premières à attaquer lesdits résidus de culture cellulosiques.

Les deux premiers facteurs limitants, l'endogénécité et la diazotrophie, ont déjà fait l'objet de technologies adaptées, notamment FR 01/15542, FR 09/05120. La sélection de bactéries fortement diazotrophes est elle aussi bien rôdée, les bases génomiques et physiologiques d'un tel phénomène étant maintenant connues. Les dynamiques des populations bactériennes du sol (BAC) au sein de la microflore du sol (MIC), et vis à vis notamment de la flore fongique du sol (FNG) sont cependant moins bien générées au profit des susdites valorisations des biomasses d'origine végétale souterraines.

Pourtant, l'*efficience carbonée* (e ; Manzoni *et al.* 2008, 2010), ou ici l'eC des BAC ($eC_{BAC}$) et/ou MIC ($eC_{MIC}$) est définie comme la quantité de carbone (ré)organisée dans la nouvelle biomasse, BAC et/ou MIC, par unité de substrat C dégradé microbiologiquement à partir des résidus de culture cellulosiques au sol. Elle ne fait donc pas expressément référence à l'azote, bien que les ratios N:C (rb ; op cit.) peuvent varier et dicter empiriquement la valeur de eC. Or, l'évaluation d'$eC_{BAC}$ ne faisant pas expressément partie de l'état de la technique, la préconisation d'inocula diazotrophes destinés aux résidus de culture cellulosiques au sol se fait un peu à l'aveuglette, les populations bactériennes in situ du même type n'étant pas nécessairement toujours supplantées par les FNG et/ou MIC. De plus, rien ne sert de tenter d'évaluer in vitro $eN_{BAC}$ et/ou $eC_{BAC}$, puisque de 90 à 99% des BAC ne sont pas cultivables in vitro. Enfin, il ne faut pas confondre efficience azotée (eN) et carbonée (eC) des BAC, les deux variables étant bien souvent opposées. $eN_{BAC}$ est une mesure du ratio de l'azote minéral assimilé dans la nouvelle biomasse BAC par unité de carbone des résidus de culture cellulosique dégradé microbiologiquement, tandis qu'$eC_{BAC}$ est une mesure du ratio du carbone ainsi assimilé par unité de carbone desdits résidus de culture cellulosiques dégradé microbiologiquement.

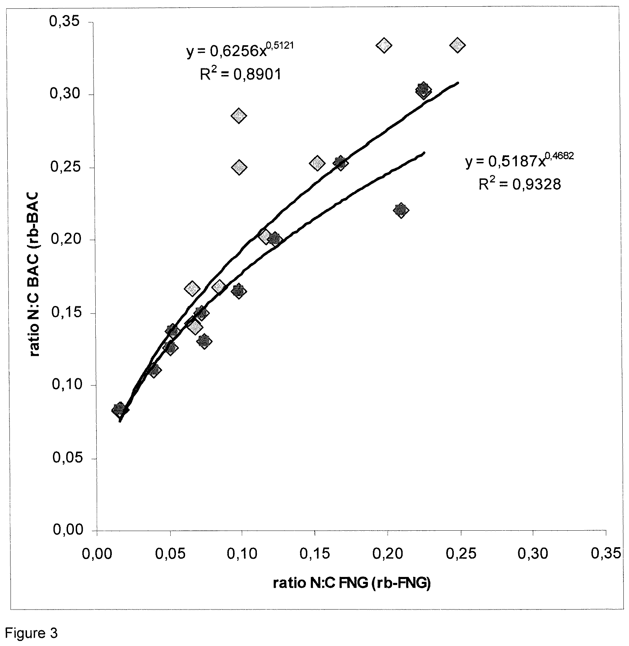

Plus particulièrement, l'évaluation d'$eC_{BAC}$ est surtout applicable à la flore azotobactérienne aérobic. En effet, leurs ratios N:C sont plus faibles en raison de l'accumulation intracellulaire (i.e. dans le cytosol) de réserves en carbone énergétique nécessaire à la production d'ATP alimentant le fonctionnement à plein régime du complexe Nif. Ce ratio N:C plutôt faible, ce rapprochant parfois de celui des décomposeurs ligno - cellulosiques du sol de ~0,10 (Manzoni et al. 2008, 2010, Cleveland et Liptzin 2007), est ainsi attribuable aux besoins énergétiques importants des Azotobacteraceae et/ou vraisemblablement à un haut taux de respiration protégeant par dissipation de l'oxygène ledit complexe Nif d'un éventuel stress oxydatif (voir Oelze 2000). L'eC, de telles Azotobacteraceae, selon l'équation (quasi)universelle de Manzoni et al. 2008 (op.cit., figure 3B), ne sera donc pas nécessairement plus faible que celles des décomposeurs attaquant en premier la résidusphère issue de l'enfouissement de résidus de culture. Or, puisque ce sont le plus souvent de telles Azotobacteraceae qui sont apportées aux dits résidus de culture, la question de leur moindre efficacité relative à celle des décomposeurs ce pose. Dans le cas d'une $eC_{BAC}$ comparable à celles des décomposeurs, l'apport de bactéries diazotrophes ne serait plus préconisé puisque les populations bactériennes, et donc Azotobacteraceae, endogènes sont en principe capables de résister par elles même sans être supplantées par celles des décomposeurs.

## LA PROBLEMATIQUE DES RELIQUATS AUTOMNAUX D'AZOTE MINERAL (rNm) EN SOLS ARABLES

On chercher généralement à réduire rNm au minimum (Claude 1990, Claude et al. 1993). Le lessivage des rNm vers les nappes phréatiques, voire leurs transformations en protoxyde d'azote ($N_2O$) émané vers l'atmosphère à titre de gaz à effet de serre sont à éviter. Pour réduire rNm, hormis l'usuelle fertilisation raisonnée, il est possible de piéger ces reliquats en cultivant des cultures intermédiaires pièges à nitrates et/ou des cultures intercalaires. Ces cultures étant par la suite détruites et incorporées au sol, l'N ainsi "piégé" peut être graduellement réintégré au cycle de l'azote du sol. Plus récemment, il est aussi question de valoriser l'habituel phénomène d'immobilisation de l'Nm par la fore microbienne du sol suite à l'apport massif de résidus de culture cellulosiques. Cette immobilisation peut en effet être augmentée par l'inoculation azotobactérienne des résidus juste avant leur enfouissement.

Or, l'immobilisation de l'azote ne pourra réduire les teneurs en Nm du sol en dessous d'un certain seuil, seuil en dessous duquel la diazotrophie de la flore azotobactérienne s'activera et ramènera rapidement de l'azote atmosphérique vers le sol. Ce phénomène, alias "$NH_4$ switch-off" attribuable à un tandem génomique dit nifLA opérera dès que la teneur en Nm de la solution du sol est d'au plus 1 mM, soit en termes d'azote environ 14 mg-Nm par kg de sol. Comme nous le verrons, cette valeur est très similaire aux susdit seuil inférieur en Nm dans les sols sujets à immobilisation de l'azote en présence de résidus de culture enfouis.

Pourtant, l'azotobactérisation des résidus de culture au sol fonctionne bel et bien même si les rNm sont bien au-delà d'une quinzaine de mg-N par kg-sol. Donc, bien que de telles inoculations azotobactériennes des résidus pailleux au sol vont nécessairement et théoriquement bénéficier d'une immobilisation quasi complète des rNm, la teneur des sols en Nm est transitoirement le plus souvent supérieure à 14 ou 15 mM. Outre ce cas extrême, comment diagnostiquer si une parcelle agronomique plus usuelle sera plus ou moins, ou non, réceptive à de telles azotobactérisations de leurs résidus de culture au sol ?

## QUEL ROLE POUR LES RELIQUATS AUTOMNAUX D'AZOTE MINERAL (RNM) DANS LE CALCUL D'EC ?

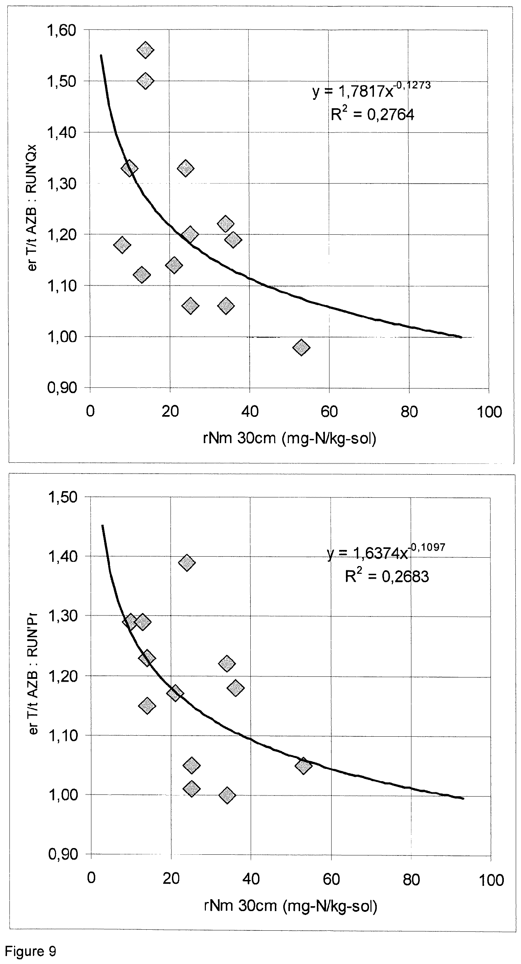

Selon Manzoni et al. 2008, l'$eC_{MIC}$ dépend de rf et rb, les ratios N:C des biomasses végétales souterraines et de la microflore du sol, respectivement ; il est raisonnable de vouloir traduire $eC_{MIC}$ en $eC_{BAC}$. Or, en raison d'une rétroaction négative de l'azote minéral, Nm, sur l'activité de la nitrogénase, d'importants reliquats automnaux d'Nm en sols arables, rNm, devrait a priori réduire l'efficacité de microorganismes, notamment et par exemple des Azotobacteraceae, appliqués sur résidus de culture cellulosique au sol (Claude et Fillion 2004). Or, comme nous le verrons, cette relation entre rNm et l'efficacité relative de telles inoculations est trop imprécise pour diagnostiquer leurs insuffisances et préconiser leurs réintroductions (infra, Figure 9). Pire, et bien qu'in extremis de très hauts taux de fertilisation N sont reconnus comme pouvant influencer les ratios N:C des BAC (eg. Yevdokimov et al.. 2005), les variations de rNm ne le sont pas, les variations de ceux-ci étant jugées trop faibles pour avoir un tel effet conséquent.

## QUELQUES BREVETS PERTINENTS ?

A ma connaissance, l'évaluation de l'efficience azotée et/ou de l'efficience carbonée bactérienne, hormis le protocole décrit dans EP 2572581 (A1).1 et FR 2995318 (A1), ne fait l'objet d'aucun protocole d'obtention et/ou d'évaluation, encore moins de préconisations palliatives en cas d'efficience azotées bactériennes jugées trop faibles. Dans le cas d'EP 12 366 001.1 et FR 12 02413, il est donc question d'un procédé d'obtention de microorganismes azotophiles, iSapro(N), comprenant une étape de mise en culture *in vitrodans* une substrat matriciel microporeux *in vitro* comprenant un certain nombre de concentrations de substrats carbonés (C) et azotés (N) assimilables par lesdits microorganismes, dont l'une est *a priori* constante à travers le substrat matriciel inerte, tel que l'agar gélosé (1 %), et l'autre diffuse graduellement établissant ainsi un gradient de ratios C/N à travers une certaine distance latérale sur laquelle pourra s'épandre radialement la biomasse iSapro(N) jusqu'à ce que cette expansion soit stoppée à un point, dNm, ou l'un ou l'autre des susdits substrats C et/ou N deviennent limitatifs. Or, cette technique nécessite la mise en culture in vitro, et donc le développement abondant de biomasse microbienne. Cette évaluation de l'efficience, azotée et non carbonée, n'intégrera donc que les biomasses ainsi cultivables in *vitro*. Très utile pour l'obtention par sélection de souches saprophytes candidates au bio - contrôle, cette technique l'est beaucoup moins si on cherche a évaluer l'azotophile de l'ensemble de la flore (azoto)bactérienne du sol, flore dont seulement une partie généralement estimée de 1 à 10% selon les auteurs, est effectivement cultivable *in vitro.*

A la lumière d'un récent rapport de recherche préliminaire français, d'autre documents peuvent aussi être *a priori* considérés comme pertinent, notamment EP2227936 A1, EP2409561 A1 et EP2223586 A1. Or, Tous ces documents concernent la biofertilisation de cultures par l'utilisation de bactéries et notamment d'*Azotobacteraceae.* L'objet de la revendication 1 de la présente invention diffère des méthodes employées dans ces documents en ce que l'efficience carbonée des populations microbiennes ($eC_{MIC}$) est calculée afin de pouvoir calculer l'efficience carbonée de la fraction bactérienne ($eC_{BAC}$) et ainsi d'obtenir l'indice de l'efficience carbonée relative $eC_{BAC}/eC_{MIC}$ (*cf. infra*).L'effet technique de cette différence permet de mesurer la capacité des flores bactériennes à résister à une trop grande domination massique des flores fongiques et microbiennes plus généralement, lors de la dégradation *in situ* des résidus de culture. Cet indice permet de savoir si les bactéries vont pouvoir "tenir tête" vis à vis de l'ensemble de la microflore microbienne du sol impliquée dans la décomposition des résidus de culture au sol et ainsi de déterminer une utilisation optimale d'inocula (azoto)bactériens dans la biofertilisation des sols.

*Sigles*

**Efficience azotée**(eN ; g-N intégrés à la nouvelle biomasse microbienne du sol / g-C de résidus de culture cellulosique dégradé par cette biomasse microbienne) : une proportion, d'azote minéral (Nm) assimilé à la nouvelle biomasse, bactérienne (**$eN_{BAC}$**), fongique (**$eN_{FNG}$**) ou microbienne (**$eN_{MIC}$**), par unité de carbone provenant de la dégradation des résidus de culture cellulosiques incorporés au sol (g-C / g-C).

**Efficience carbonée**(eC ; g-C intégrés à la nouvelle biomasse microbienne du sol / g-C de résidus de culture cellulosique dégradé par cette biomasse microbienne) : une proportion, de carbone intégré à la nouvelle biomasse, bactérienne (**$eC_{BAC}$**), fongique(**$eC_{FNG}$**), ou microbienne (**$eC_{MIC}$**), par unité de carbone provenant de la dégradation des résidus de culture cellulosiques incorporés au sol (g-C / g-C).

**Indice d'efficience carbonée relative** ($eC_{BAC}/eC_{MIC}$) : le rapport entre l'eC des fractions bactérienne (BAC) et microbienne (MIC) du sol, ou encore plus simplement et généralement, **efficience relative,** azotée (**erN**) ou carbonée (**erC**) : ratio des eN, ou eC, des BAC, FNG ou MIC les une par rapport aux autres. Eg. $eC_{BAC}/eC_{MIC}$.

**Efficacité relative** (er) : ratio entre une valeur paramétrique avec (traitement) et sans (témoin) un quelconque traitement, par exemple ici l'azotobactérisation par inoculation des résidus de culture pailleux au sol (AZB).

**AZB** : azotobactérisation à l'aide de populations azotobactériennes réintroduites, généralement par pulvérisation liquide des résidus de culture et/ou végétaux, cellulosique, voire avantageusement pailleux, au sol.

**BAC :** fraction, ou composante, bactérienne de la microflore (MIC) du sol. Celle-ci peut être déterminée physico - chimiquement, ou encore déduite à partir de MIC et ratio fongique : bactérien (rFB). Elle peut aussi être séparée physiquement, par centrifugation d'une suspension aqueuse du sol (cf. Bakken 1985), ou chimiquement, sans nécessairement impliquer la mise en culture des cellules bactériennes qui pourrait biaiser malencontreusement sa composition au profit des bactéries les plus copiotrophes.

**FNG** : fraction, ou composante, fongique de la microflore (MIC) du sol. Celle-ci peut être déterminée physico - chimiquement, ou encore déduite à partir de MIC et ratio fongique : bactérien (rFB). Elle peut aussi en principe être séparée physiquement selon Bakken 1985.

**MIC** : ensemble de la microflore, y compris les fractions fongique (FNG) et bactérienne, du sol

**$rCN_{sol}$** : ratio carbone sur azote du sol, ou plus précisément de la matière organique du sol.

**rf :** ratio N:C des résidus de culture incorporés au sol : rapports entre les teneurs en N et C des résidus de cultures et/ou tout substrat carboné pouvant être décomposés in situ par la flore microbienne du sol (cf. Manzoni et al. 2008/2010).

**rb** ratios N:C des MIC (**$rb_{MIC}$**), FNG (**$rb_{FNG}$**) et/ou BAC (**$rb_{BAC}$**) décomposeurs impliqués dans la décomposition in situ des résidus de culture et/ou tout substrat carboné (cf. Manzoni et al. 2008/2010).

**rNm** : reliquats d'azote minéral (mg-N / kg-sol) toujours présent dans un sol arable après la récolte d'une culture en prévision de l'incorporation de résidus de culture cellulosiques.

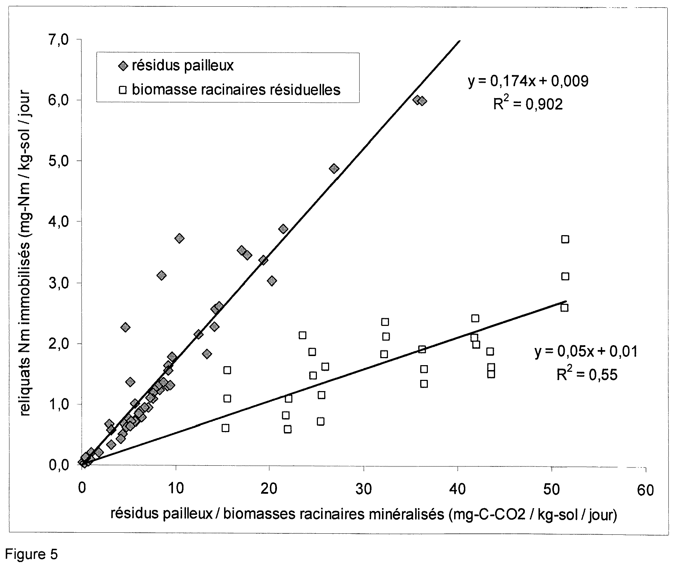

**rFB** : ratio, massique, des composantes fongique (FNG) et bactériennes (BAC) de la microflore (MIC) du sol. Ce ratio peut être obtenus empiriquement via l'équation proposée par Fierer et al. 2009 (figure 5).

DIVULGATION DE L'INVENTION

Problème technique

Le problème technique à résoudre peut donc être formulée comme étant l'optimisation de la biofertilisation par des(azoto)bactéries. Bien que la détermination de l'$eC_{MIC}$ soit bien connue (Manzoni et al. 2008, 2010), aucun document de l'art antérieur ne divulgue le calcul d'$eC_{BAC}$ ainsi que de l'indice de l'efficience carbonée relative $eC_{BAC}$ / $eC_{MIC}$, sans parler de l'application d'un tel indice pour optimiser la biofertilisation. Comme pour toute valorisation de biomasses souterraines d'origine végétale, la préconisation des AZB sur résidus de culture cellulosiques fait problème. Elle n'intègre pas une détermination d'$eC_{BAC}$, ou même à ce stade d'$eN_{BAC}$ régissant la capacité qu'ont les BAC, et accessoirement les Azotobacteraceae, de résister en nombre sans être supplantées une fois réintroduite en sols arables et confrontées à une domination des FNG. Il en résulte que l'utilisation de tels inocula (azoto)bactériens au sens de Claude et Fillion 2004 est dans une certains nombre de cas peu ou pas effective, ou du moins non optimale ; dans de tels cas, l'impasse de l'inoculation aurait été une préconisation plus judicieuse.

De plus, l'évaluation de la l'efficience carbonée des populations bactériennes ($eC_{BAC}$), voire plus généralement microbiennes ($eC_{MIC}$)ne doit pas dépendre de leurs *cultivabilitéin vitro.* En effet, de 90 à 99% des cellules bactériennes spécifiques appartenant à ces populations n'étant pas ainsi cultivables *in vitro,* l'évaluation d'$eC_{BAC}$ via de telles mises en culture serait grossièrement non représentative de l'état *in situ* de ces populations.

Solution technique

L'efficience carbonée des *décomposeursin situ,* lire - la microflore du sol, des résidus de culture peut a priori être calculée via l'équation de Manzoni *et al.* 2008 (figure 3A) ;

$$eC_{MIC} = 0,45 \, [rf]^{\,0,76}$$

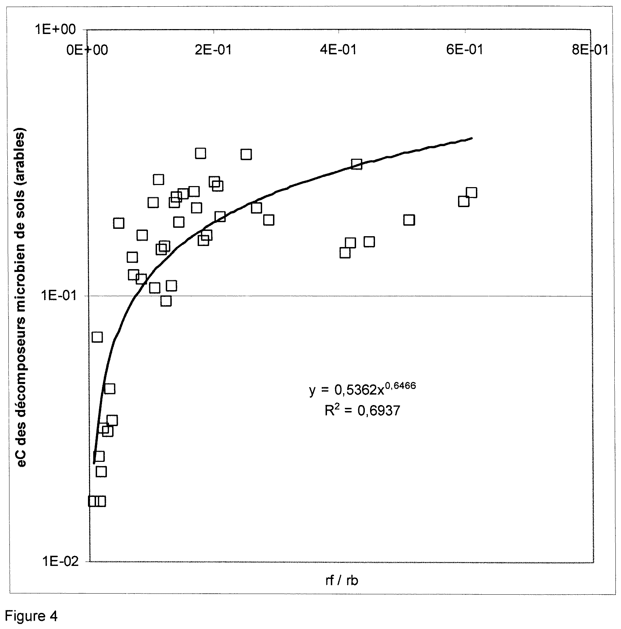

où rf est le ratio N:C des résidus de culture, pailleux notamment, enfouis. Plus utilement, il est aussi possible de prévoir $eC_{MIC}$ selon la l'équation suivante (voir ici Figure 4) inspirée de celle rapportée dans Manzoni *et al.* 2008 (figure 3B) ;

$$eC_{MIC} = 0,54 \, [rf/rb]^{\,0,65}$$

où cette fois-ci rb et le ratio N:C non pas nécessairement des susdits décomposeurs, mais des *consommateurs,* soit directement des résidus de culture, soit indirectement de leurs métabolites. Cette deuxième équationest donc en principe applicable plus spécifiquement aux BAC pour les quelles il est maintenant possible, en principe, de calculer une valeur $eC_{BAC}$, comparable à $eC_{MIC}$. On obtient ainsi <u>deux</u> valeurs, $eC_{MIC}$ et $eC_{BAC}$, comparables mais pas nécessairement égales. Encore faut-il pouvoir obtenir $rb_{BAC}$ sans mise en culture *in vitro.*

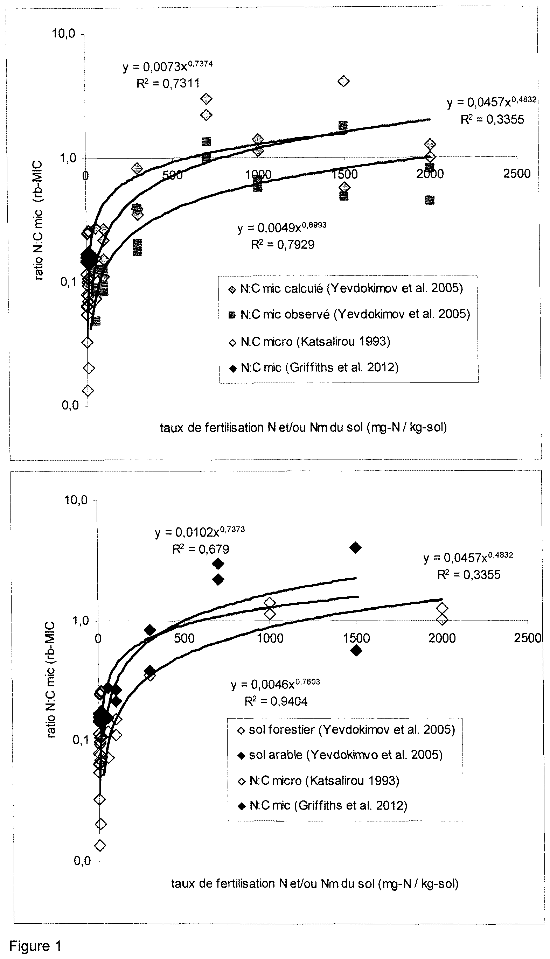

Pour ce faire, l'invention combine quatre (4) équations permettant de transformer de simples données agronomiques et pédologiques en diagnostiques préconisant ou non l'utilisation d'inocula microbiologiques sur des résidus de culture cellulosiques (pailleux) au sol. On doit dans un premier temps déterminer le ratio N:C de la microflore du sol (rbMIC) selon l'importance des reliquats automnaux d'azote minéral selon une équation empirique inspirée des données, entres autres, de Yevdokimov *et al.* 2005 (Figure 1) ;

$$rb_{MIC} = \mathbf{a} \times rNm^{\mathbf{b}} \qquad\qquad (1)$$

La prémisse de Manzoni *et al.* 2008 selon laquelle la valeur de rb (i.e. N:C des décomposeurs du sol) est essentiellement inchangeable et de 0,10 semble être défendable. En effet, j'ai recompilé (Figure X) des ratios N:C microbiens à partir de données $N_{mic}$ et $C_{mic}$ publiées dans Cleveland et Liptzin 2007 provenant de 135 sites forestiers et prairies ; statistiquement la moyenne est de 0,126 +/- 0,048 et la médiane de 0,120. Cependant, la pente m de la droite ;

$$N_{mic} = mC_{mic} + c$$

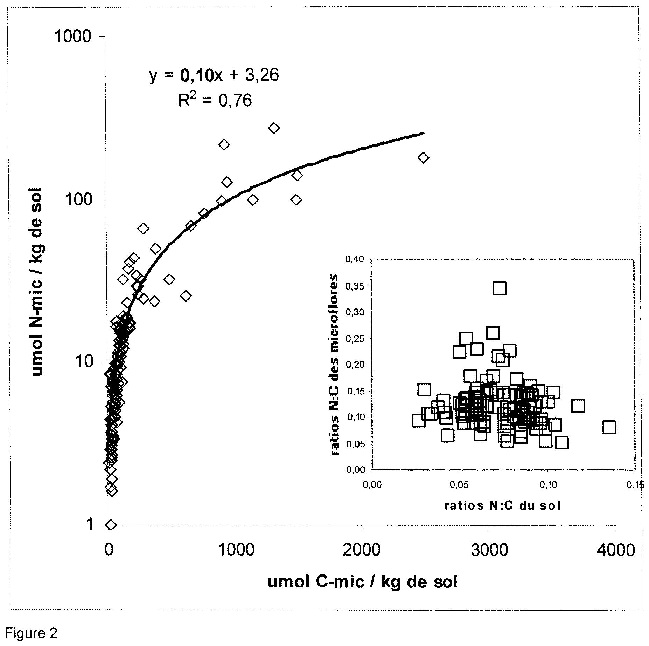

est elle plus révélatrice puisque m est exactement égale à 0,10, soit la valeur rb préconisée indépendamment par Manzoni *et al.* 2008 (op. cit., figure 3A). Pareillement, ce qu'il y a de contre intuitif, ce que les ratios Nmic:Cmic de sont pas du tout corrélés avec les ratio Nsol:Csol (en abîme, Figure 2), ce qui confirme le constat que le ratio N:C du cytosol, et donc $eC_{MIC}$ ne dépends que du ratio N:C (rf) des résidus de culture, voire $rb_{MIC}$.

A noter ici en quoi la présente invention va à l'encontre de la règle établie selon laquelle $rb_{MIC}$ est relativement inchangeable à ~ 0,10 (Manzoni *et al.* 2008). Dans un agrosystème, cette valeur de 0,10 est effectivement, en moyenne à travers le temps voire la campagne est approximativement correcte ; voir les données de Cleveland et Liptzin 2008 rapportées graphiquement à la Figure 2. Or, $rb_{MIC}$, et donc rbBAC et rbFNG, vont transitoirement à travers l'interculture entre le semis et la *sortie d'hiver* (i.e. début du printemps) varier selon l'rNm. C'est du moins la prémisse de la présente invention. A noter encore une fois comme ce constat bouscule l'ordre établie par la technique ; cette dépendance de rbMIC sur rNm est ici posée en extrapolant une régression (Figure 1) basée sur des données, des taux de fertilisation azotée en kg-N par hectare, de une à deux ordre de grandeurs supérieures aux rNm normalement (usuellement) trouvés dans les sols arables. Cette extrapolation est ici pour le moins audacieuse.

Il faut par la suite, selon le ratio C:N de la matière organique du sol ($rCN_{sol}$), calculer le ratio pondéral flore fongique (FNG) : flore bactérienne (BAC), rFB. Il s'agit ici d'une équations empirique adaptée de Fierer *et al.* 2009 reliant rCNsol aux nombres de copies du gène ARNm 16S bactérien par rapport à celui du gène ARNm 18S fongique ;

$$rFB = a \times \exp(rCN_{sol} \times b) \qquad (2)$$

Il fallut cependant transformer ces ratios 18S:16S en ratios massique. Pour ce faire j'ai établi le ratio entre le nombre de nucléotides des ARNm 16S (~1500) / 18S (~1900) = 0,7895, ainsi que le ratio entre les volumes habituels de cellules BAC et FNG, soit 4,19 = 314 = 0,0133. Pour transformer le rFB arénique en rFB massique il s'agit donc simplement de le multiplier par 0,0133 x 0,7895 ≈ 0,01. Connaissant rFB il est ainsi possible de calculer à partir de rbMIC la valeur de $rb_{FNG}$ ainsi ; $rb_{FNG} = (2 \times rb_{MIC}) / (1 + rFB)$. A l'aide de cette valeur de $rb_{FNG}$, et compte tenu de la progression non linéaire de $rb_{BAC}$ par rapport à $rb_{FNG}$, on calcul $rb_{BAC}$ ainsi (Figure 3) ;

$$rb_{BAC} = a \times [rb_{FNG}]^b \qquad (3)$$

Enfin, connaissant rb pour MIC et BAC, il est possible de calculer l'efficience carbonée, eC, de ces composantes BAC et MIC de la microflore du sol selon l'équation suivante (Figure 4) ;

$$eC = a \times [rf/rb]^b \qquad (4)$$

A noter que rf est ici le ratio N:C des résidus de culture cellulosique, voire pailleux, incorporés au sol ; l'efficience carbonée relative, $eC_{BAC}/eC_{MIC}$, ne sera donc pas affectée par rf, ce qui est étonnant compte tenu de l'application de l'invention à l'évaluation de l'état métabolique des BAC et MIC en présence de tels résidus de culture. Or, à bien y voir, les AZB particulièrement efficientes (e ; Manzoni *et al.* 2008) vont, à partir d'une quantité de substrat carboné donnée produire plus de biomasse et du coup assimiler plus d'azote minéral Nm que les AZB qui le sont moins.

A ce stade de développement, les valeurs paramétriques les plus réalistes pour ce quatre (4) équations sont présentés au Tableau 1 ;

**Tableau 1** : Valeurs empiriques des coefficients a et b des fonctions empiriques (1) à (4)

| formule empirique | a | b | référence data |
|---|---|---|---|
| **(1)** $rb_{MIC} = f[rNm]$ | 0,0068 | 0,7336 | Yevdokimov *et al.* 2005 |
| **(2)** $rFB = f[rCN]$ | 0,008 | 0,11 | Fierer *et al.* 2009 |
| **(3)** $rb_{BAC} = f[rb_{FNG}]$ | 0,52 | 0,47 | Strickland et Rousk 2010 |
| **(4)** $eC_{MIC} = F[rf/rb_{MIC}]$ ; $eC_{BAC} = f[rf/rb_{BAC}]$ | 0,54 | 0,65 | Manzoni *et al.* 2008/2010 |

Une AZB plus efficiente aura de besoin de plus d'Nm afin de produire d'autant plus de biomasse au rapport N:C contant. Ce faisant, elle minéralisera (respirera) d'autant plus de carbone sous forme de dioxyde de carbone. Or, il s'avère que le ratio entre l'immobilisation des rNm et la minéralisation du C des résidus de culture est pour les composeurs, essentiellement MIC e FNG, est relativement constant pur un type de résidus de culture donnée (eg. Figure 5). Il est donc possible pour un type de substrat C in situ d'établir le ration N:C préféré de tels décomposeurs à partir de courbes (quasi)universelles. Ce par rapport à ces ratios N:C (rb) des décomposeurs du sol que l'on considère ici comme normale (de référence) que l'on compare les ratios N:C des AZB obtenues, soit par extraction et mesure directes, soit plus avantageusement par calcul à partir des rNm de des rCN du sol.

La solution technique proposée prévoit donc la détermination d'$eC_{BAC}$ et $eC_{MIC}$ afin de les comparer rationnellement, $eC_{BAC} / eC_{MIC}$, et d'obtenir ainsi une eC relative (erC) indicatrice de la capacité des flores BAC, y compris donc des AZB endogènes réintroduites via la pulvérisation des résidus de culture au sol, à résister à une trop grande domination massique, des flores FNG, et MIC plus généralement, lors de la dégradation in situ desdites résidus de culture.

Il s'agit donc de savoir si les BAC vont dans l'ensemble pouvoir "tenir tête" via à vis de l'ensemble de la microflore microbienne du sol impliquée dans la décomposition des résidus de culture (pailleux) au sol. Une efficacité relative, ou *efficience* (e) au sens de Manzoni *et al.* 2008/2010 eBAC/eMIC ≥ 1,00 indique que la flore (azoto)bactérienne est en ce sens in situ suffisamment efficiente et qu'il n'est pas impératif de la renforcer par inoculation des résidusphères, Or, il s'avère que cette efficacité relative est surtout fonction du ratio C:N (rCN) du sol, mais pas de celui des résidus de culture.

Concrètement, il s'agit donc d'un procédé d'évaluation de l'efficience carbonée (eC) des populations microbiennes du sol en présence de biomasses d'origines végétales souterraines et de reliquats automnaux d'azote minéral (rNm), et de préconisation de l'utilisation de microorganismes réintroduits par inoculation pouvant valoriser ces biomasses, par transformation de données agronomiques et pédologiques en un indice de l'efficience carbonée relative de la fraction bactérienne (BAC) de la microflore (MIC) du sol, $eC_{BAC}/eC_{MIC}$, caractérisé en ce qu'il comprend les étapes suivantes ;

> une première étape d'échantillonnage du sol afin d'obtenir un échantillon représentatif de la zone ou du volume de sol dans lequel seront incorporés les biomasses d'origines végétales ;

> une deuxième étape de détermination des reliquats d'azote minérale (rNm) de l'échantillon afin de déterminer en fonction de rNm le ratio N:C ($rb_{MIC}$) de la flore microbienne du sol (MIC) ;

> une troisième étape de détermination du ratio carbone/azote de l'échantillon de sol ($rCN_{sol}$) afin de déterminer en fonction de $rCN_{sol}$ le ratio entre les fractions fongique (FNG) et bactérienne (BAC) du sol (rFB) ;

> une quatrième étape de détermination du ratio N:C ($rb_{BAC}$) de la fraction bactérienne (BAC) de la flore microbienne (MIC) du sol en fonction de $rb_{MIC}$ une fois cette valeur $rb_{MIC}$ ajusté selon le rFB ;

> une cinquième étape de détermination de l'efficience carbonée (eC) de la fraction BAC ($eC_{BAC}$) et de MIC ($eC_{MIC}$) ;

> une sixième étape de détermination de l'indice de l'efficience carboné relative, $eC_{BAC}/eG_{MIC}$, de la fraction BAC par rapport à celle de MIC ;

> une septième étape de détermination de l'indice de l'efficience carbonée relative, $eC_{SAC}/eC_{MIC}$, c'est-à-dire le rapport entre l'eC des fractions bactérienne (BAC) et microbiennes (MIC) du sol, et l'appréciation de cet indice par rapport à un seuil critique compris dans une intervalle de confiance de 0,75 et 1,00, plus particulièrement entre les valeurs de 0,92 et 0,98, et avantageusement d'environ 0,95 indiquant une niveau d'efficacité relative de 1,0 par rapport à un témoin non inoculé *d'une utilisation de microorganismes réintroduits par inoculation valorisant de telles biomasses souterraines d'origines végétales.*

Les biomasses d'origines végétales sont des résidus de culture cellulosiques, avantageusement pailleux, tandis que les microorganismes réintroduits par inoculation pouvant valoriser ces biomasses sont bactériens, et avantageusement azotobactériens, c'est-à-dire pouvant réduire biologiquement et en présence d'oxygène l'azote diatomique, $N_2$.

La détermination de rbMIC en fonction de rNm ce fait à l'aide d'une fonction du type,

$$rb_{MIC} = \mathbf{a}[rNm]^{\mathbf{b}},$$

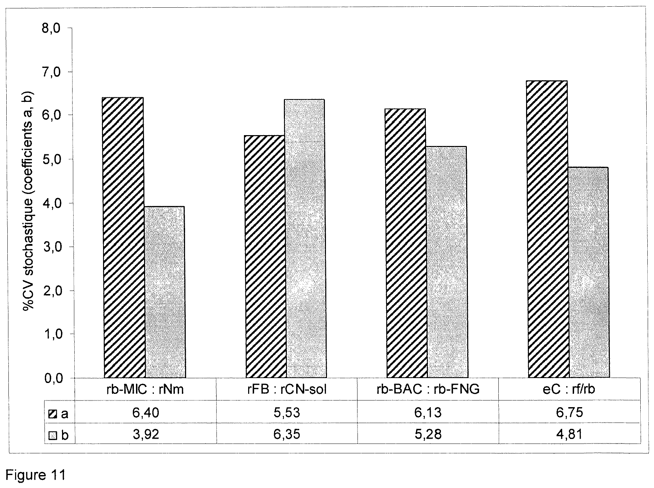

les coefficients a et b pouvant avoir des valeurs de 0,0068 +/- 5 à 7%, et 0,7336 +/- 4 à 6%, respectivement ; les écarts de 4 à 7% selon le cas sont issus de simulations stochastiques (Figure 11). La détermination de rFB en fonction de $rCN_{sol}$ ce fait à l'aide d'une fonction du type,

$$rFB = \mathbf{a}[\exp(\mathbf{b}(rCN_{sol}))],$$

les coefficients a et b pouvant avoir des valeurs de 0,008 +/- 5 à 7%, et 0,11 +/- 4 à 6%, respectivement. La détermination de $rb_{BAC}$ en fonction de $rb_{MIC}$ une fois cette valeur $rb_{MIC}$ ajustée selon le rFB ce fait à l'aide d'une fonction du type,

$$rb_{BAC} = \mathbf{a}[rb_{FNG}]^{\mathbf{b}},$$

lorsque $rbFNG = (2 \times rb_{MIC}) / (rFB +1)$ ; les coefficients a et b peuvent avoir des valeurs de 0,52 +/-5 à 7%, et 0,47 +/- 4 à 6%, respectivement. Enfin, la détermination de l'efficience carbonée (eC) des fractions BAC ($eC_{BAC}$) et MIC ($eC_{MIC}$) ce fait à l'aide d'une fonction du type,

$$eC = \mathbf{a}[rf/rb]^{\mathbf{b}},$$

les coefficients a et b pouvant avoir des valeurs de 0,54 +/- 5 à 7%, et 0,65 +/- 4 à 6%, respectivement.

L'appréciation de cet indice de l'efficience carbonée relative, $eC_{BAC}/eC_{MIC}$, peut aussi ce faire par rapport à une seuil critique compris à l'intérieur d'intervalles de *prédiction* dont les bornes inférieures indiquent des valeurs critiques comprises entre 0,75 et 0,92, plus particulièrement entre 0,80 et 0,90, et avantageusement de 0,85 indiquant une niveau d'efficacité relative de 1,05 à 1,1 *d'une utilisation de microorganismes réintroduits par inoculation valorisant de telles biomasses souterraines d'origines végétales.* Ces intervalles de prédiction dont les bornes inférieures indiquent des valeurs critiques comprises entre 0,75 et 0,92, plus particulièrement entre 0,80 et 0,90, et avantageusement de 0,85, sont établies selon une fonction du type,

$$Y = aX^b +/- t_n \times S \times racine(1/n + (X_o - \dot{X})^2 / \Sigma X^2)$$

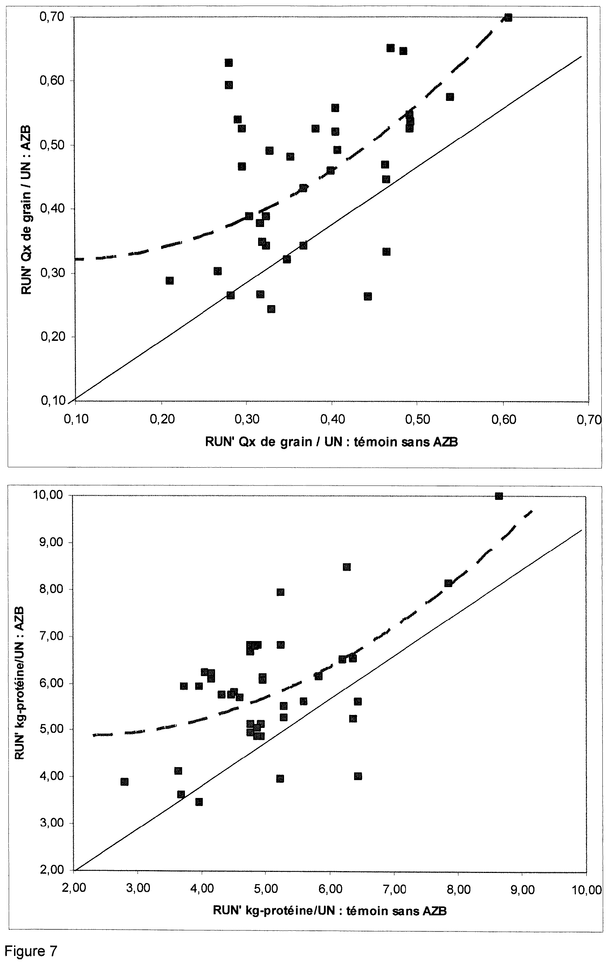

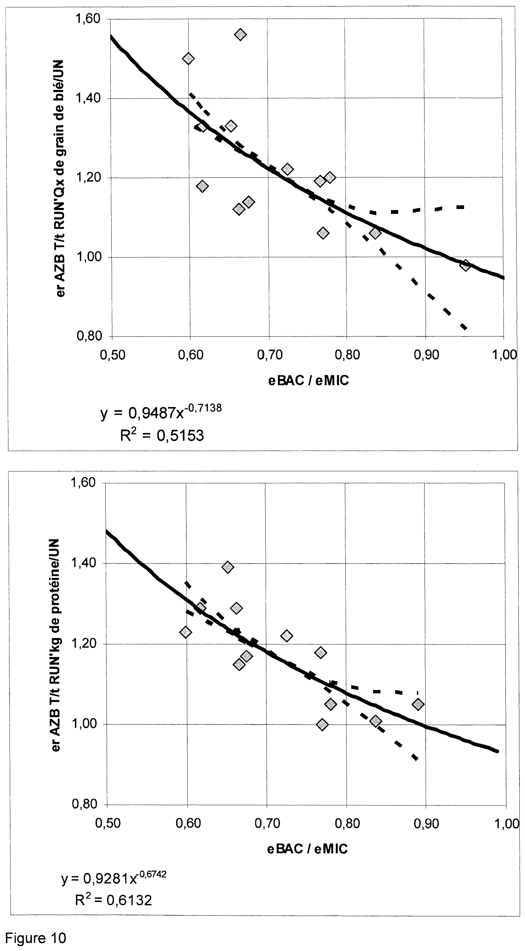

où Y est l'indice d'efficience carbonée relative (Figure 10), a et b sont les coefficients des fonctions en puissance à la Figure 10 reliant ledit indice à l'efficacité relative de d'AZB, i.e. l'azotobactérisation des résidus de culture au sol (Figure 7), $t_\alpha$ est la valeur critique de t pour un niveau de probabilité $\alpha$, n le nombre d'observations (Tableau 3), $X_o$ la valeur de l'observation, $\dot{X}$ la moyenne des n observations, et $\Sigma X^2$ la somme de leurs carrés- Ici $\alpha$ est fixée à 0,01 et t0,01 à 2,65 ou 2,68 selon le nombre d'observations, 12 ou 13 selon le cas (voir Tableau 3).

*Avantages apportés et activité inventive*

L'invention permet de savoir si une azotobactérisation des résidus de culture au sol, de type AZB™ par exemple, sera probablement efficace, ou non. Dans le cas d'une efficacité probable, une telle valorisation des résidus de culture au sol sera préconisée. Pour ce faire, seules quelques données agro - pédologiques, soit ici les rNm et le rCN du sol, sont nécessaires.

La détermination de $rb_{MIC}$ à partir des valeurs de rNm n'est pas habituelle. L'homme du métier, à la lumière des données produites par Yevdokimov *et al.* 2005 cherchera à déterminer $rb_{MIC}$ à partir du taux de fertilisation N, ces taux étant chez Yevdokimov *et al.* 2005 très élevés, bien au-delà des taux appliqués sur grandes cultures agronomiques, et près de 100 fois supérieurs dans bien des cas au rNm. Pourtant, ici, ce type de fonction (Figure 2) ce sont avérées en ce sens fort utiles est étonnamment suffisamment précises malgré des extrapolations graphiques a priori audacieuses.

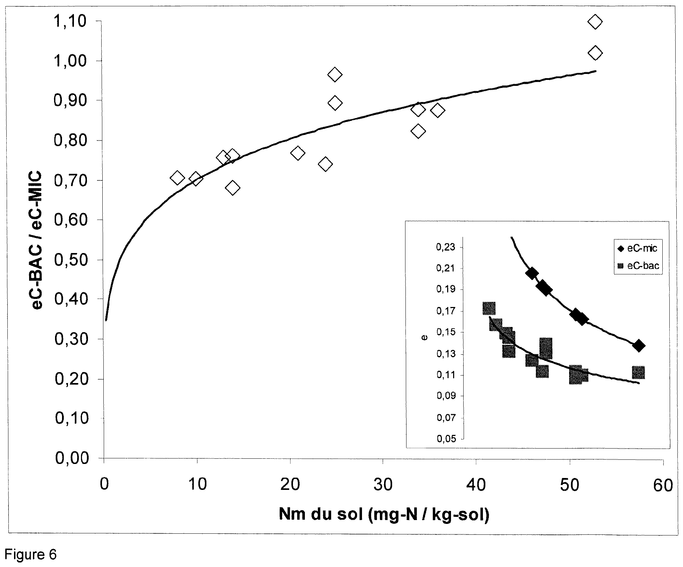

La détermination des $rb_{BAC}$ n'est pas ici une simple transposition de $rb_{MIC}$, mais une proportion de $rb_{MIC}$ tenant compte de rFB lui-même fonction du rCN du sol. Cela dit, j'ai ajusté $rb_{BAC}$ selon $rb_{FNG}$, la progression de rbBAC selon rbFNG n'étant pas linéaire (Figure 1). Or, c'est surtout cette non linéarité qui explique que la dégression de eC est plus rapide pour FNG (Figure 6), que le ratio eBAC / eFNG se rapproche graduellement de 1,00, préconisant ainsi l'impasse de AZB. A noter que cette implication du ratio C:N du sol (rCN) dans la détermination d'$eC_{MIC}$ et $eC_{BAC}$ à l'aide des rapports rf:rb (Figure 4) est étonnante vue la non corrélation entre rbMIC et/ou rbBAC et rCN (Figure 2, en abîme).

Enfin, appliquer détermination de e (efficience carbonée ; g-C intégrés à la biomasse / g-C de substrat (résidus de culture) dégradé) à l'évaluation des population azotobactériennes du sol est a priori contre intuitive. Ces populations AZB sont normalement évaluer selon leur efficience diazotrophe (mg-$N_2$ intégrés à la biomasse par réduction enzymatique /g-C de substrat (résidus de culture) dégradé). De même, l'efficacité d'une azotobactérisation des résidus de culture au sol est surtout perçue par l'homme du métier comme le fait d'une moindre rétroaction négative de l'opéron nifLA en présence de rNm faible, plutôt que d'une efficience carbonée de BAC relativement moins atténuée que celle de FNG (Figure 7).

**DESCRIPTION DES DESSINS ET FIGURES**

**Figure 1** : Progression des ratios N:C de la flore microbienne du sol (MIC) selon la teneur des sols en azote minéral (Nm ; mg-N / kg-sol). Diverses fonctions empiriques ont été établies à partir des données de Yevdokimov *et al.* 2005, Katsalirou 1993 et Griffiths *et al.* 2012. A noter qu'il s'agit ici surtout de ratio N:C selon le taux de fertilisation (Yevdokimov *et al.* 2005) ; les données rNm de Katsalirou 1993 et Griffiths *et al.* 2012 ayant été superposées pour en évaluer la concordance.

**Figure 2** : Corrélation linéaire entre les teneurs en azote microbien (umol-Nmic / kg de sol) et carbone microbien (umol-Cmic / kg de sol) pour divers types de sol forestiers, arables et paries (données de Cleveland et Liptzin 2007). A noter l'échelle logarithmique de l'axe Y, ainsi que la pente de la droite égale à 0,10, soit la valeur N:C (quasi)universelle pour les décomposeurs in situ proposé par Manzoni *et al.* 2008 (op. cit., figure 3A). En abîme, le nuage de points non corrélés correspondant aux ratios N:C des microflore du sol par rapport à celles des N:C des sols d'où elles proviennent.

**Figure 3** : Progression des ratios N:C de la fraction bactérienne (BAC) du sol en fonction de ceux de la fraction fongique (FNG). Les deux jeux de données empiriques proviennent pour l'essentiel de Strickland et Rousk 2010.

**Figure 4** : Corrélation en puissance de l'efficience carboné (eC ; taux d'incorporation dans la nouvelle biomasse microbienne du carbone provenant de la dégradation des résidus de culture ; données de Manzoni *et al.* 2008, figure 3) par rapport aux ratios rf/rb, i.e. les ratios des N:C des résidus de culture (rf) par rapport à ceux des consommateurs (rb) de ces résidus de culture et/ou de leurs métabolites carbonés. Dans le cas des décomposeurs in situ des résidus de culture, rb est essentiellement fixé à 0,10 ; dans le cas des bactéries, et plus particulièrement des azotobactéries (AZB) du sol, rb sera déterminé empiriquement- Ces deux valeurs de eC sont donc comparables.

**Figure 5** : Corrélations linéaires entre les taux (mg de $C-CO_2$ par kg de sol et par jour) de minéralisation des résidus de culture pailleux et de biomasses racinaires résiduelles (prairies) et les taux d'immobilisation de l'azote minéral Nm (mg de Nm par kg de sol et par jour). A noter une certaine constance spécifique à chaque type de résidus en cours de dégradation. (Données de diverses sources ; Recous *et al.* 1998, Mary *et al.* 1998, Mary *et al.* 1996, Garnier *et al.* 2003 et Barrett et Burke 2000).

**Figure 6** : Modélisation - Progression de l'efficience carbonée relative, eBAC /eMIC, de la faction bactérienne du sol, BAC, par rapport à celle de la microflore du sol dans son ensemble, MIC, selon l'importance des reliquats d'azote minéral (rNm ; mg-N /kg-sol) ; En abîme, la dégression des efficiences carbonés selon rNm pour BAC et MIC. Les efficiences carbonés ont été calculées selon l'équation proposée par Manzoni *et al.* 2008 et les valeurs rb (ratios N:C) prédites via les équations à la Figure 2. Voir les Tableaux 1 et 3 pour les valeurs paramétriques et les simulations numériques.

**Figure 7** : Rapport entre les progression des rendements unitaires (RUN') agronomiques (Qx de grain /UN) et protéiques (kg de protéine dans le grain / UN) avec et sans l'azotobactérisation (AZB) des résidus de culture au sol. Ces données, croisées avec les rNm pour chacune des parcelles (données rNm au Tableau 3) serviront à la validation de l'invention. A noter que parcelles (points variables) avec AZB™ sont le plus souvent en haut et à gauche de la diagonale 1:1, d'où des augmentations hautement significatives des RUN' agronomiques et protéiques de l'ordre de 15 à 25%.

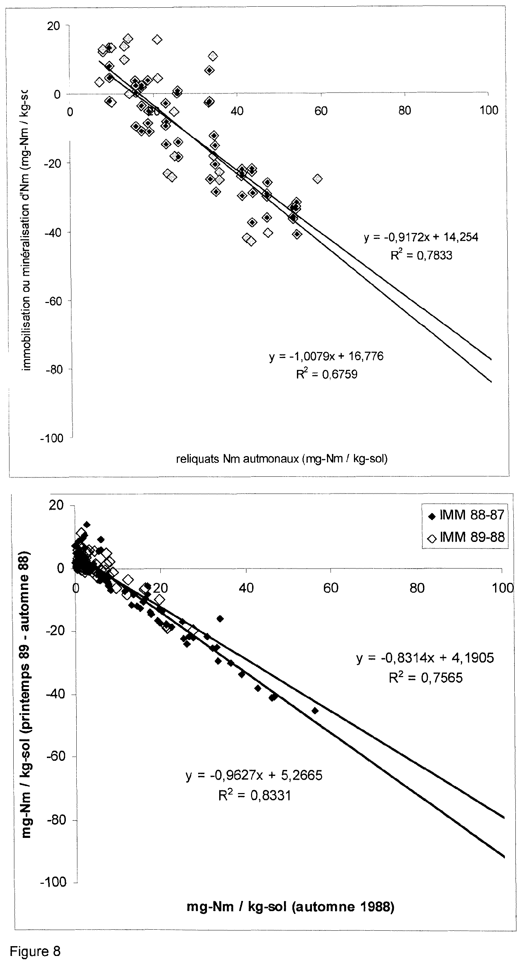

**Figure 8:** Relation entre la quantité d'azote Nm immobilisée (valeurs négatives) et/ou minéralisée (valeurs positives) si les rNmsont faibles, et la quantité de tels reliquats d'Nm. Le graphe du haut résume les données de Claude 1997 en serre avec résidus pailleux, et in situ avec blé d'hiver en 2010-2011 sur résidus de culture pailleux enfouis en présence de blé tendre d'hiver ; le graphe du bas résume le données de Claude 1990 et Claude *et al.*1993, *in situ* (Québec) sur résidus de maïs grain en présence de cultures intercalaires *Poaceae* et *Fabaceae.* A noter que ladite immobilisation /minéralisation est égale à 0,00 lorsque les reliquats automnaux sont d'environ 14à 16 mg-Nm par kg de sol ; cette valeur est à mettre en relation avec les 1 $mM-NH_4$ (14 mg-N par litre ou kg de sol) généralement considéré comme inhibiteurs (*ammonium switch-off* ; Hartmann *et al.* 1986) de la nitrogénase.

**Figure 9** : Validation - Les efficacités relative d'une azotobactérisation de résidus de culture pailleux au sol (rCN 80) en terme de rendements unitaires ajustés (RUN') pondéraux (Qx de blé d'hiver/ UN) ou protéique (kg protéine / UN) sont en puissance fonction inversés des reliquats d'azote minéral automnaux (rNm ; mg-N /kg-sol) ; on pourrait croire a priori que cette fonction inverse révèle une certaine rétroaction négative de Nif via nifLA par l'ammonium (*alias* - "*ammonium switch off*").

**Figure 10** : Validation - Les efficacité relative d'une azotobactérisation de résidus de culture pailleux au sol (rCN 80) en terme de rendements unitaires ajustés (RUN') pondéraux (Qx de grains de blé d'hiver / UN) ou protéique (kg protéine grain / UN) sont fonction en puissanceinversée du ratio $eC_{BAC}$ / $eC_{MIC}$. Ces fonctions tenant compte des ratios C:N du sol (rCN) sont plus précises ($R^2$ ~60%) et indiquent clairement que si $eC_{BAC}$ atteint~95% d'$eC_{MIC}$ l'efficacité relative (er) de telles azotobactérisations (AZB) ne dépassera guère 1,00. Pour rendre l'interprétation plus pratique, j'ai inclus des intervalles de

confiance ($t_{0,01}$) de part et d'autre ces régressions. A noter qu'ainsi, dès le ratio $eC_{BAC}$ / $eC_{MIC}$ supérieur à ~0,85, l'er d'AZB ne dépassera probablement pas 1,00 ; une telle azotobactérisation des résidus de culture au sol n'est donc plus préconisée.

**Figure 11** : Paramétrage stochastique des coefficients **a** et **b** (cf. Tableau 1) des équations (1), (2), (3) et (4). A noter une certaine robustesse du modèle rbe dans la limite de variations de ces coefficients de 4 à 7 % par rapport au valeurs proposées issues de régressions non linéaires des données de Yevdokimov et al. 2005 (éq. 1), Fierer et al. 2009 (éq. 2), Strickland et Rousk 2010 (éq. 3) et Manzoni et al. 2008 et 2010 (éq. 4). Nb. Ce paramétrage stochastique est ici qu'exemplaire et la réalisation de l'invention pourrait de réaliser avec des coefficients variant au-delà de ces bornes de 4 à 7%.

## REALISATION DE L'INVENTION

Le diagnostic que nous devons poser est de savoir si cette efficience azotée des AZB du sol leur permet de ne pas être supplantées par la microflore de décomposeurs en présence de résidus de culture enfouis. En d'autres mots, l'eC des populations bactériennes du sol (BAC) est-elle comparable à celle des populations desdits décomposeurs microbiens du sol (MIC), ou du moins suffisante pour leur tenir tête au cours de la décomposition de la résidusphère, pailleuse notamment? Dans le cas contraire, il sera préconisé d'inoculer lesdites résidusphères, par exemple par azotobactérisation au sens de Claude et Fillion 2004.

On peut assimiler $eC_{AZB}$ à $eC_{BAC}$, ce qui est raisonnable, les populations de bactéries diazotrophes étant ubiquitaires dans les sols arables, et les Azotobacteraceae, faisant partie des gamma protéobactéries elles aussi très répandues en sols arables. Nous allons par la suite calculer $eC_{BAC}/eC_{MIC}$. La comparaison de ces deux valeurs d'eC équivaut à comparer l'efficience carbonée des BAC et AZB à celles des décomposeur in situ de résidus de culture au ratio N:C donné ;

- si $eC_{BAC} < eC_{MIC}$, les BAC pourraient donc être supplantés lors de la décomposition desdits résidus de culture, et le renforcement de leurs populations dans la résidusphère serait donc préconisée, d'où par exemple l'utilité d'une azotobactérisation (AZB) des résidus de culture pailleux au sol ;
- si $eC_{BAC} \geq eC_{MIC}$, les BAC seraient donc suffisamment efficientes pour tenir tête aux décomposeurs, elles ne risquerait pas d'être supplantées par l'ensemble de MIC, voire plus particulièrement sa composante FNG ; l'utilité d'AZB serait dans un tel cas marginale voire nulle.

## APPLICATIONS ET VALIDATIONS EN SITUATIONS AGRONOMIQUES

L'invention doit permettre d'évaluer a priori l'efficacité relative d'une (azoto)bactérisation de résidus de culture, avantageusement pailleux, au sol qu'en fonction des reliquats d'azote automnaux (rNm) et du ratio C:N du sol ($rCN_{sol}$). Pour valider un tel diagnostique, j'ai utilisé des données agronomiques provenant d'une campagne d'essai d'inocula azotobactériens appliqués sur de tels résidus de culture au sol en prévision d'une culture de blé d'hiver dans la région Midi-Pyrénées (France). Les rNm ont été évalués en octobre 2010 sur ces treize (13) parcelles agricoles (peu après le semis des blés).

A la récolte, les deux bandes essais voisines, avec (T) et sans (t) l'azotobactérisation, ont été échantillonnées de trois à quatre fois de manière appariée afin d'obtenir une cinquantaine de valeurs estimées de leurs rendements agronomiques (pondéreux ; Qx de grain / hectare) et protéiques (kg de protéine du grain / hectare). Connaissant le taux de fertilisation azotée, TUN (kg N-fertilisant / ha), j'ai calculé les rendements unitaires agronomiques et/ou protéiques (RUN ; Qx ou kg de protéine (Pr) de blé produits par unités d'azote (kg-N / ha)). Ces RUN ont été ajustés selon l'importance de TUN (RUN' ; idem), les rendements unitaires décroissant normalement lorsque TUN augmente, indépendamment d'un éventuel effet azotobactérien. L'efficacité relative par rapport aux témoins (t) de telles azotobactérisation (T) des résidus de culture est rapportée graphiquement à la Figure 7.

Or, nous connaissons aussi pour ces bandes essai les reliquats d'azote automnaux (rNm ; Figure 8) que nous pouvons ainsi mettre en relation avec les efficacité relatives T/t en termes de RUN' (Figure 9). Tel qu'attendu, cette efficacité relative décroît avec l'augmentation de rNm, a priori ont pourrait croire en raison d'une rétroaction négative de la nitrogenase par rNm (Tableau 2) ;

**Tableau 2** : Efficacités relatives moyennes de AZB™ (récolte 2011 ; +/- l'écart type) en terme de rendements unitaires (**RUN'**) agronomiques (**Qx** de grain / UN) et protéiques (kg de **Pr**otéine / UN) selon la gamme (n = nombre d'observation ; cf. Tableau 3) de reliquats d'azote minéral (rNm)

| gamme de rNm | ------------- efficacité relative T/t moyenne d'AZB ------------- | |
|---|---|---|
| (mg-N/kg-sol) | RUN'**Qx** | RUN'**Pr** |
| de 8 à 15 (n=10) | 1,34 +/- 0,10 | 1,24 +/- 0,04 |
| de 16 à 25 (n = 8) | 1,18 +/- 0,06 | 1,16 +/- 0,09 |
| de 26 à plus de 50 (n = 8) | 1,11 +/- 0,06 | 1,11 +/- 0,05 |

Or, nous pouvons faire mieux, et aller au-delà de simple constat que l'AZB des résidus de culture au sol pourraient être plus efficaces in situ si les teneurs des sols en Nm sont plutôt faibles. En effet, à l'aide des équations 1 à 4 intégrées par exemple à une simple feuille de calcul Excel™ (Tableau 3), et connaissant rCN, nous pouvons calculer les valeurs de rbBAC et rbMIC nécessaires à l'estimation des eCBAC et eCMIC, les comparer rationnellement (eCBAC / eCMIC), et enfin les mettre en relation avec les susdites er T/t d'AZB (Figure 7).

Graphiquement, ces relations (Figure 10) sont bien plus précises et explicatives que celles à la Figure 9. Elles nous indiquent plus clairement que dès une un ration $eC_{BAC}$ / $eC_{MIC}$ d'au plus 0,90, les BAC du sol étant par elles même capables de tenir tête aux MIC, l'azotobactérisation (AZB) des résidus de culture au sol (RCsol) ne sera pas en soi très efficace ; mieux vaudra dans ce cas préconiser l'impasse de telles AZB. Étant donnée l'imperfection de ces courbes à ce stade de développement de l'invention (Figure 10), je leur ai ajouté des intervalles de prédictions ($t_{0,01}$). Le tracé de ces intervalles inférieures (pointillés ; Figure 10) croise la courbe de régression à environ une er d'AZB (T/t) 1,00 lorsque $eC_{BAC}$ / $eC_{MIC}$ n'est plus que d'environ 0,80 à 0,85, soit par extrapolation lorsque l'er d'AZB (T/t) est d'environ 1,08 à 1,10. En termes de rNm (Figure 9), de telles erAZB peuvent donc, selon les $rCN_{SOL}$, être compromises par des rNm somme tout assez habituels en situations agronomiques de l'ordre de 40 à 60 mg-Nm /kg-sol. L'incidence de préconisations dictant l'impasse des telles AZB sera donc a priori non négligeable.

Enfin, la robustesse de ces déterminations par modélisation a été évalué. Par modulation stochastique, il est établi que les coefficients empiriques **a** et **b** utilisés ici est listés au Tableau 1 peuvent varier de 5 à 7% dans le cas du coefficient **a**, et de 3 à 5% dans le cas du coefficient **b** (Figure 11). A ce stade de développement expérimental de l'invention, on peut donc affirmer que des déterminations de ratios $eC_{BAC}$ / $eC_{MIC}$ à l'aide des coefficients compris entres ces bornes mèneront essentiellement aux mêmes préconisations.

## Références

Bakken, LR. 1985. Séparation and Purification of Bacteria from Soil. Appl. Environ. Microbiol. 49 : 1482-1487

Barrett et Burke 2000. Potential nitrogen immobilization in grassland soils across a soil organic matter gradient. Soil Biol. Biochem. 32 : 1707-1716

Claude, PP 1990. Effet des cultures intercalaires dans le maïs grains sur le rendement en grain, la qualité édaphique, et la teneur en azote inorganique des sols. Thèse de doctorat (Ph.D.) 1900, Université McGill, Montréal, Québec (Canada).(108 pp).

Claude, PP et al. 1993. Effet du labour printanier et de cultures intercalaires de trèfle rouge sur le rendement du maïs grain. Can. J. Plant Sci. 73 : 37-49

Claude, PP 1997. Valorisation des résidus de culture et biofertilisation. Mémoire, Diplôme de recherche universitaire (DRU), École nationale supérieure d'agronomie, INPT (Toulouse, novembre 1997, France),

Claude, PP et L Fillon. 2004. Effet de l'apport d'un inoculum bactérien aux résidus de culture de maïs-grain au sol sur le rendement et la qualité de blés d'hiver panifiables en France. Agrosol. (depuis - Agrosolutions) 15 (1) : 23-29

Cleveland, CC et D Liptzin 2007. C:N:P stoichiometry in soil: is there a "Redfield ratio" for the microbial biomass? Biogeochemistry 85:235-252

Fierer et al. 2009. Global patterns in belowground communities. Ecology Letters, (2009) 12: 1-12

Garnier et al. 2003. Modelling carbon and nitrogen dynamics in a bare soit with and without strawincorporation. European Journal of Soil Science, September 2003, 54, 555-568

Griffiths et al. 2012. C:N:P stoichiometry and nutrient limitation of the soil microbial biomass in a grazed grassland site under experimental P limitation or excess. Ecological Processes 2012, 1:6

Katsalirou 1993. Microbial community and enzyme activities in prairie soil ecosystems under different management. M. Sc. in Soil Science, Aristotle University of Thessaloniki, Thessaloniki, Greece 1998

Manzoni et al. 2008. The global stoichiometry of litter nitrogen mineralisation. Science 321, 684 (2008)

Manzoni et al. 2010. Stoichiometric controls on carbon, nitrogen, and phosphorus dynamics in decomposing litter. Ecological Monographs, 80(1), 2010, pp. 89-106

Mary et al. 1998. A model for calculating nitrogen fluxes in soil using 15N tracing. Soil Biol. Biochem. 30 : 1963-1979

Mary et al. 1996. Interactions between décomposition of plant residues and nitrogen cycling in soil. Plant and Soil 181:71-82

Oelze 2000. Respiratory protection of nitrogenase in Azotobacter species: is a widely held hypothesis unequivocally supported by experimental evidence? FEMS Microbiology Reviews 24 : 321-333

Recous et al. 1999. In situ changes in gross N transformations in bare soil after addition of straw. Soil Biol. Biochem. 31 : 119-133

Strickland et Rousk 2010. Considering fungal:bacterial dominance in soils e Methods, controls, and ecosystem implications. Soil Biology & Biochemistry 42 (2010) 1385 -1395

Yevdokimov et al. 2005. Nitrogen Immobilization by Soil Microorganisms Depending on Nitrogen Application Rates. Eurasian Soil Science, Vol. 38, No. 5, 2005, pp. 516-523. Translated from Pochvovedenie, No. 5, 2005, pp. 581-589.

**Tableau 3:** Données empiriques et calculées ayant servies à la détermination des efficacité relative $eC_{BAC}$ / $eC_{MIC}$, associées à une série de valeurs d'efficacités relatives, Traité/témoin, de AZB en termes de rendements unitaires (RUN') agronomiques (**Qx** de grain/UN) et protéiques (kg de **P**rotéine/UN). A noter que les $rb_{BAC}$ sont calculés à partir des $rb_{FNG}$ et non à partir des $rb_{MIC}$ directement fonction de rNm.

| unités → | na | mg-N/kg-sol | na | na | na (ARNm) | na | na | na | rf/rb = eC (g-$C_{BIOM}$ / g-C Rcsol) | | | | | |
|---|---|---|---|---|---|---|---|---|---|---|---|---|---|---|
| id | N:C Rcsol (rf) | rNm | $rb_{MIC}$ | $rCN_{SOL}$ | rFB | rFB | $rb_{FNG}$ | $rb_{BAC}$ | $eC_{MIC}$ | $eC_{FNG}$ | $eC_{BAC}$ | $eC_{BAC}$/$eC_{MIC}$ | T/t RUN'Qx | T/t RUN'Px |
| 1 | 0,0125 | 8 | 0,0313 | 15,00 | 0,042 | 4,17 | 0,0121 | 0,0653 | 0,2976 | 0,5514 | 0,1844 | 0,6195 | 1,18 | |
| 2 | 0,0125 | 14 | 0,0471 | 8,50 | 0,020 | 2,04 | 0,0310 | 0,1017 | 0,2279 | 0,2990 | 0,1383 | 0,6067 | 1,50 | 1,23 |
| 3 | 0,0125 | 10 | 0,0368 | 13,00 | 0,033 | 3,34 | 0,0170 | 0,0765 | 0,2676 | 0,4429 | 0,1663 | 0,6216 | 1,33 | 1,29 |
| 4 | 0,0125 | 24 | 0,0700 | 6,00 | 0,015 | 1,55 | 0,0549 | 0,1330 | 0,1762 | 0,2063 | 0,1161 | 0,6589 | 1,33 | 1,39 |
| 5 | 0,0125 | 14 | 0,0471 | 12,50 | 0,032 | 3,16 | 0,0226 | 0,0877 | 0,2279 | 0,3671 | 0,1523 | 0,6681 | 1,56 | 1,15 |
| 6 | 0,0125 | 21 | 0,0635 | 8,91 | 0,021 | 2,13 | 0,0405 | 0,1152 | 0,1878 | 0,2514 | 0,1274 | 0,6785 | 1,14 | 1,17 |
| 7 | 0,0125 | 13 | 0,0446 | 13,00 | 0,033 | 3,34 | 0,0206 | 0,0838 | 0,2361 | 0,3908 | 0,1568 | 0,6642 | 1,12 | 1,29 |
| 8 | 0,0125 | 34 | 0,0904 | 6,50 | 0,016 | 1,64 | 0,0686 | 0,1476 | 0,1493 | 0,1786 | 0,1085 | 0,7270 | 1,22 | 1,22 |
| 9 | 0,0125 | 36 | 0,0942 | 8,25 | 0,020 | 1,98 | 0,0632 | 0,1420 | 0,1453 | 0,1883 | 0,1113 | 0,7660 | 1,19 | 1,18 |
| 10 | 0,0125 | 34 | 0,0904 | 9,00 | 0,022 | 2.15 | 0,0573 | 0,1356 | 0,1493 | 0,2007 | 0,1146 | 0,7680 | 1,06 | 1,00 |
| 11 | 0,0125 | 25 | 0,0721 | 12,57 | 0,032 | 3,19 | 0,0344 | 0,1068 | 0,1728 | 0,2795 | 0,1339 | 0,7749 | 1,20 | 1,05 |
| 12 | 0,0125 | 25 | 0,0721 | 15,00 | 0,042 | 4,17 | 0,0279 | 0,0967 | 0,1728 | 0,3203 | 0,1428 | 0,8262 | 1,06 | 1,01 |
| 13 | 0,0125 | 53 | 0,1252 | 12,50 | 0,032 | 3,16 | 0,0601 | 0,1387 | 0,1208 | 0,1946 | 0,1130 | 0,9353 | 0,98 | 1,05 |

**Revendications**

1. Procédé d'évaluation de l'efficience carbonée (eC) des populations microbiennes du sol en présence de biomasses d'origines végétales souterraines et de reliquats automnaux d'azote minéral (rNm), et de préconisation de l'utilisation de microorganismes réintroduits par inoculation pouvant valoriser ces biomasses, par transformation de données agronomiques et pédologiques en un indice de l'efficience carbonée relative de la fraction bactérienne (BAC) de la microflore (MIC) du sol, $eC_{BAC}/eC_{MIC}$, **caractérisé en ce qu'**il comprend les étapes suivantes ;

   ➢ une première étape d'échantillonnage du sol afin d'obtenir un échantillon représentatif de la zone ou du volume de sol dans lequel seront incorporés les biomasses d'origines végétales ;
   ➢ une deuxième étape de détermination des reliquats d'azote minérale (rNm) de l'échantillon afin de déterminer en fonction de rNm le ratio N:C ($rb_{MIC}$) de la flore microbienne du sol (MIC) ;
   ➢ une troisième étape de détermination du ratio carbone/azote de l'échantillon de sol ($rCN_{sol}$) afin de déterminer en fonction de $rCN_{sol}$ le ratio entre les fractions fongique (FNG) et bactérienne (BAC) du sol (rFB) ;
   ➢ une quatrième étape de détermination du ratio N:C ($rb_{BAC}$) de la fraction bactérienne (BAC) de la flore microbienne (MIC) du sol en fonction de $rb_{MIC}$ une fois cette valeur $rb_{MIC}$ ajusté selon le rFB ;
   ➢ une cinquième étape de détermination de l'efficience carbonée (eC) de la fraction BAC ($eC_{BAC}$) et de MIC ($eC_{MIC}$) ;
   ➢ une sixième étape de détermination de l'indice de l'efficience carboné relative, $eC_{BAC}/eC_{MIC}$, de la fraction BAC par rapport à celle de MIC ;
   ➢ une septième étape de détermination de l'indice de l'efficience carbonée relative, $eC_{BAC}/eC_{MIC}$, c'est-à-dire le rapport entre l'eC des fractions bactérienne (BAC) et microbiennes (MIC) du sol, et l'appréciation de cet indice par rapport à un seuil critique compris dans une intervalle de confiance de 0,75 et 1,00, plus particulièrement entre les valeurs de 0,92 et 0,98, et avantageusement d'environ 0,95 indiquant une niveau d'efficacité relative de 1,0 par rapport à un témoin non inoculé *d'une utilisation de microorganismes réintroduits par inoculation valorisant de telles biomasses souterraines d'origines végétales.*

2. Procédé d'évaluation de l'efficience carbonée (eC) des populations microbiennes du sol en présence de biomasses d'origines végétales souterraines et de reliquats automnaux d'azote minéral (rNm), et de préconisation de l'utilisation de microorganismes réintroduits par inoculation pouvant valoriser ces biomasses selon la première revendication **caractérisé en ce que** les biomasses d'origines végétales sont des résidus de culture cellulosiques, avantageusement pailleux.

3. Procédé d'évaluation de l'efficience carbonée (eC) des populations microbiennes du sol en présence de biomasses d'origines végétales souterraines et de reliquats automnaux d'azote minéral (rNm), et de préconisation de l'utilisation de microorganismes réintroduits par inoculation pouvant valoriser ces biomasses selon une quelconque des revendications précédentes **caractérisé en ce que** les microorganismes réintroduits par inoculation pouvant valoriser ces biomasses sont bactériens, et avantageusement azotobactériens, c'est-à-dire pouvant réduire biologiquement et en présence d'oxygène l'azote diatomique, $N_2$.

4. Procédé d'évaluation de l'efficience carbonée (eC) des populations microbiennes du sol en présence de biomasses d'origines végétales souterraines et de reliquats automnaux d'azote minéral (rNm), et de préconisation de l'utilisation de microorganismes réintroduits par inoculation pouvant valoriser ces biomasses selon une quelconque des revendications précédentes **caractérisé en ce que** la détermination de $rb_{MIC}$ en fonction de rNm ce fait à l'aide d'une fonction du type $rb_{MIC} = \mathbf{a}[rNm]^{\mathbf{b}}$.

5. Procédé d'évaluation de l'efficience carbonée (eC) des populations microbiennes du sol en présence de biomasses d'origines végétales souterraines et de reliquats automnaux d'azote minéral (rNm), et de préconisation de l'utilisation de microorganismes réintroduits par inoculation pouvant valoriser ces biomasses selon la revendication précédente **caractérisé en ce que** les coefficients $\underline{a}$ et $\underline{b}$ ont des valeurs de 0,0068 +/- 5 à 7%, et 0,7336 +/- 4 à 6%, respectivement.

6. Procédé d'évaluation de l'efficience carbonée (eC) des populations microbiennes du sol en présence de biomasses d'origines végétales souterraines et de reliquats automnaux d'azote minéral (rNm), et de préconisation de l'utilisation de microorganismes réintroduits par inoculation pouvant valoriser ces biomasses selon une quelconque des revendications précédentes **caractérisé en ce que** la détermination de rFB en fonction de $rCN_{sol}$ ce fait à l'aide d'une fonction du type rFB (massique) = $\mathbf{a}[\exp(\mathbf{b}(rCNsol))]$.

7. Procédé d'évaluation de l'efficience carbonée (eC) des populations microbiennes du sol en présence de biomasses d'origines végétales souterraines et de reliquats automnaux d'azote minéral (rNm), et de préconisation de l'utilisation de microorganismes réintroduits par inoculation pouvant valoriser ces biomasses selon la revendication précédente **caractérisé en ce que** les coefficients a et b ont des valeurs de 0,008 +/- 5 à 7%, et 0,11 +/- 4 à 6%, respectivement.

8. Procédé d'évaluation de l'efficience carbonée (eC) des populations microbiennes du sol en présence de biomasses d'origines végétales souterraines et de reliquats automnaux d'azote minéral (rNm), et de préconisation de l'utilisation de microorganismes réintroduits par inoculation pouvant valoriser ces biomasses selon une quelconque des revendications précédentes **caractérisé en ce que** la détermination de $rb_{BAC}$ en fonction de $rb_{MIC}$ une fois cette valeur $rb_{MIC}$ ajustée selon le rFB ce fais à l'aide d'une fonction du type $rb_{BAC} = \mathbf{a}[rb_{FNG}]^{\mathbf{b}}$, et que $rb_{FNG} = (2(rb_{MIC})) / (rFB + 1)$.

9. Procédé d'évaluation de l'efficience carbonée (eC) des populations microbiennes du sol en présence de biomasses d'origines végétales souterraines et de reliquats automnaux d'azote minéral (rNm), et de préconisation de l'utilisation de microorganismes réintroduits par inoculation pouvant valoriser ces biomasses selon la revendication précédente **caractérisé en ce que** les coefficients a et b ont des valeurs de 0,52 +/- 5 à 7%, et 0,47 +/- 4 à 6%, respectivement.

10. Procédé d'évaluation de l'efficience carbonée (eC) des populations microbiennes du sol en présence de biomasses d'origines végétales souterraines et de reliquats automnaux d'azote minéral (rNm), et de préconisation de l'utilisation de microorganismes réintroduits par inoculation pouvant valoriser ces biomasses selon une quelconque des revendications précédentes **caractérisé en ce que** la détermination de l'efficience carbonée des fractions BAC ($eC_{BAC}$) et MIC ($eC_{MIC}$) de la microflore du sol ce fait à l'aide d'une fonction du type $eC = \mathbf{a}[rf/rb]^{\mathbf{b}}$.

11. Procédé d'évaluation de l'efficience carbonée (eC) des populations microbiennes du sol en présence de biomasses d'origines végétales souterraines et de reliquats automnaux d'azote minéral (rNm), et de préconisation de l'utilisation de microorganismes réintroduits par inoculation pouvant valoriser ces biomasses selon la revendication précédente **caractérisé en ce que** les coefficients a et b ont des valeurs de 0,54 +/- 5 à 7%, et 0,65 +/- 4 à 6%, respectivement.

12. Procédé d'évaluation de l'efficience carbonée (eC) des populations microbiennes du sol en présence de biomasses d'origines végétales souterraines et de reliquats automnaux d'azote minéral (rNm), et de préconisation de l'utilisation de microorganismes réintroduits par inoculation pouvant valoriser ces biomasses selon une quelconque des revendications précédentes **caractérisé en ce que** l'indice de l'efficience carbonée relative, $eC_{BAC}/eC_{MIC}$, c'est-à-dire le rapport entre l'eC des fractions bactérienne (BAC) et microbienne (MIC) du sol, et l'appréciation de cet indice par rapport à une seuil critique compris à l'intérieur d'intervalles de prédiction dont les bornes inférieures indiquent des valeurs critiques comprises entre 0,75 et 0,92, plus particulièrement entre 0,80 et 0,90, et avantageusement de 0,85 indiquant une niveau d'efficacité relative de 1,05 à 1,1 *d'une utilisation de microorganismes réintroduits par inoculation valorisant de telles biomasses souterraines d'origines végétales.*

13. Procédé d'évaluation de l'efficience carbonée (eC) des populations microbiennes du sol en présence de biomasses d'origines végétales souterraines et de reliquats automnaux d'azote minéral (rNm), et de préconisation de l'utilisation de microorganismes réintroduits par inoculation pouvant valoriser ces biomasses selon la revendication précédente **caractérisé en ce que** les intervalles de prédiction dont les bornes inférieures indiquent des valeurs critiques comprises entre 0,75 et 0,92 sont établies selon une fonction du type,

$$Y = aX^b +/- t_n \times S \times racine(1/n + (X_o - \ddot{X})^2 / \Sigma X^2)$$

où Y est l'indice d'efficience carbonée relative, a et b sont les coefficients des fonctions en puissance reliant l'indice à l'efficacité relative de l'(azoto)bactérisation des résidus de culture au sol, $t_\alpha$ est la valeur critique de t pour un niveau de probabilité $\alpha$, n le nombre d'observations , $X_o$ la valeur de l'observation, X la moyenne des n observations, et $\sum X^2$ la somme de leurs carrés.

**Patentansprüche**

1. Verfahren zur Bewertung der Kohlenstoff-Effizienz (eC) mikrobieller Populationen des Bodens in Anwesenheit unterirdischer Pflanzenbiomassen und herbstlicher mineralischer Stickstoffrückstände (rNm) und zur Empfehlung der Verwendung von durch Inokulation wiedereingeführten Mikroorganismen, die diese Biomassen verwerten können, durch Verarbeitung landwirtschaftlicher und bodenkundlicher Daten in einem Index der relativen Kohlenstoff-Effizienz der Bakterienfraktion (BAC) der Mikroflora (MIC) des Bodens, $eC_{BAC}/eC_{MIC}$, das dadurch charakterisiert ist, dass es die folgenden Schritte umfasst:

> einen ersten Schritt mit einer Bodenprobe, um eine repräsentative Probe der Bodenzone oder des -volumens zu erhalten, in die die pflanzliche Biomasse eingearbeitet wird;

> einen zweiten Schritt zur Bestimmung der mineralischen Stickstoffrückstände (rNm) der Probe, um in Abhängigkeit von rNm das N/C-Verhältnis ($rb_{MIC}$) der mikrobiellen Bodenflora (MIC) zu bestimmen;

> einen dritten Schritt zur Bestimmung des Kohlenstoff-/Stickstoff-Verhältnisses der Bodenprobe ($rCN_{sol}$), um das Verhältnis zwischen der Pilz- (FNG) und der Bakterienfraktion (BAC) des Bodens (rFB) zu bestimmen;

> einen vierten Schritt zur Bestimmung des N/C-Verhältnisses ($rb_{BAC}$) der Bakterienfraktion (BAC) der mikrobiellen Bodenflora (MIC) als Funktion von $rb_{MIC}$, wenn dieser $rb_{MIC}$-Wert gemäß rFB eingestellt wird;

> einen fünften Schritt zur Bestimmung der Kohlenstoff-Effizienz (eC) der BAC-Fraktion ($eC_{BAC}$) und der MIC-Fraktion (eCMIC);

> einen sechsten Schritt zur Bestimmung des Index der relativen Kohlenstoff-Effizienz $eC_{BAC}/eC_{MIC}$ der BAC- im Vergleich zur MIC-Fraktion;

> einen siebten Schritt zur Bestimmung des Index der relativen Kohlenstoff-Effizienz $eC_{BAC}/eC_{MIC}$, das heißt des Verhältnisses zwischen dem eC der bakteriellen (BAC) und mikrobiellen (MIC) Fraktionen des Bodens, und der Aufwertung dieses Index in Bezug auf eine kritische Schwelle innerhalb eines Konfidenzintervalls von 0,75 bis 1,00, insbesondere zwischen den Werten von 0,92 und 0,98, und am besten etwa 0,95, was auf ein relatives Effizienzniveau von 1,0 im Vergleich zu einer nicht okulierten Kontrolle *für eine Verwendung von durch Inokulation wiedereingeführten Mikroorganismen, die diese unterirdischen Biomassen verwerten können, hinweist.*

2. Verfahren zur Bewertung der Kohlenstoff-Effizienz (eC) mikrobieller Populationen des Bodens in Anwesenheit unterirdischer Pflanzenbiomassen und herbstlicher mineralischer Stickstoffrückstände (rNm) und zur Empfehlung der Verwendung von durch Inokulation wiedereingeführten Mikroorganismen, die diese Biomassen verwerten können, gemäß dem ersten Anspruch, der dadurch charakterisiert ist, dass es sich bei der Biomasse pflanzlichen Ursprungs um Ernterückstände aus Zellulose, am besten um Stroh, handelt.

3. Verfahren zur Bewertung der Kohlenstoff-Effizienz (eC) mikrobieller Populationen des Bodens in Anwesenheit unterirdischer Pflanzenbiomassen und herbstlicher mineralischer Stickstoffrückstände (rNm) und zur Empfehlung der Verwendung von durch Inokulation wiedereingeführten Mikroorganismen, die diese Biomassen verwerten können, gemäß einem der vorhergehenden Ansprüche, der dadurch charakterisiert ist, dass die durch Inokulation wiedereingeführten Mikroorganismen, die diese Biomassen verwerten können, Bakterien, am besten Azotobakterien sind, das heißt, dass diese biologisch und in Anwesenheit von Sauerstoff zweiatomigen $N_2$ reduzieren können.

4. Verfahren zur Bewertung der Kohlenstoff-Effizienz (eC) mikrobieller Populationen des Bodens in Anwesenheit unterirdischer Pflanzenbiomassen und herbstlicher mineralischer Stickstoffrückstände (rNm) und zur Empfehlung der Verwendung von durch Inokulation wiedereingeführten Mikroorganismen, die diese Biomassen verwerten können, gemäß einem der vorhergehenden Ansprüche, der dadurch charakterisiert ist, dass die Bestimmung von $rb_{MIC}$ als Funktion von $r_{Nm}$ unter Verwendung einer Funktion vom Typ $rb_{MIC} = a[rNm]^b$ erfolgt.

5. Verfahren zur Bewertung der Kohlenstoff-Effizienz (eC) mikrobieller Populationen des Bodens in Anwesenheit unterirdischer Pflanzenbiomassen und herbstlicher mineralischer Stickstoffrückstände (rNm) und zur Empfehlung der Verwendung von durch Inokulation wiedereingeführten Mikroorganismen, die diese Biomassen verwerten können, gemäß dem vorhergehenden Anspruch, der dadurch charakterisiert ist, dass die Koeffizienten $\underline{a}$ und $\underline{b}$ Werte von 0,0068 +/- 5 bis 7 %, beziehungsweise 0,7336 +/- 4 bis 6 % haben.

6. Verfahren zur Bewertung der Kohlenstoff-Effizienz (eC) mikrobieller Populationen des Bodens in Anwesenheit unterirdischer Pflanzenbiomassen und herbstlicher mineralischer Stickstoffrückstände (rNm) und zur Empfehlung der Verwendung von durch Inokulation wiedereingeführten Mikroorganismen, die diese Biomassen verwerten können, gemäß einem der vorhergehenden Ansprüche, der dadurch charakterisiert ist, dass die Bestimmung von rFB als Funktion von $rCN_{sol}$ mithilfe einer Funktion vom Typ rFB (massebezogen) = $\mathbf{a}[exp(\mathbf{b}(rCNsol))]$ erfolgt.

**7.** Verfahren zur Bewertung der Kohlenstoff-Effizienz (eC) mikrobieller Populationen des Bodens in Anwesenheit unterirdischer Pflanzenbiomassen und herbstlicher mineralischer Stickstoffrückstände (rNm) und zur Empfehlung der Verwendung von durch Inokulation wiedereingeführten Mikroorganismen, die diese Biomassen verwerten können, gemäß dem vorhergehenden Anspruch, der dadurch charakterisiert ist, dass die Koeffizienten $\underline{a}$ und $\underline{b}$ Werte von 0,008 +/- 5 bis 7 %, beziehungsweise 0,11 +/- 4 bis 6 % haben.

**8.** Verfahren zur Bewertung der Kohlenstoff-Effizienz (eC) mikrobieller Populationen des Bodens in Anwesenheit unterirdischer Pflanzenbiomassen und herbstlicher mineralischer Stickstoffrückstände (rNm) und zur Empfehlung der Verwendung von durch Inokulation wiedereingeführten Mikroorganismen, die diese Biomassen verwerten können, gemäß einem der vorhergehenden Ansprüche, der dadurch charakterisiert ist, dass die Bestimmung von $rb_{BAC}$ als Funktion von rbMIC, nachdem dieser $rb_{MIC}$-Wert gemäß rFB eingestellt wurde, mithilfe einer Funktion vom Typ $rb_{BAC} = \mathbf{a}[rb_{FNG}]^b$ erfolgt und dass $rb_{FNG} = (2(rb_{MIC})) / (rFB + 1)$.

**9.** Verfahren zur Bewertung der Kohlenstoff-Effizienz (eC) mikrobieller Populationen des Bodens in Anwesenheit unterirdischer Pflanzenbiomassen und herbstlicher mineralischer Stickstoffrückstände (rNm) und zur Empfehlung der Verwendung von durch Inokulation wiedereingeführten Mikroorganismen, die diese Biomassen verwerten können, gemäß dem vorhergehenden Anspruch, der dadurch charakterisiert ist, dass die Koeffizienten $\underline{a}$ und $\underline{b}$ Werte von 0,52 +/- 5 bis 7 % beziehungsweise 0,47 +/- 4 bis 6 % haben.

**10.** Verfahren zur Bewertung der Kohlenstoff-Effizienz (eC) mikrobieller Populationen des Bodens in Anwesenheit unterirdischer Pflanzenbiomassen und herbstlicher mineralischer Stickstoffrückstände (rNm) und zur Empfehlung der Verwendung von durch Inokulation wiedereingeführten Mikroorganismen, die diese Biomassen verwerten können, gemäß einem der vorhergehenden Ansprüche, der dadurch charakterisiert ist, dass die Bestimmung der Kohlenstoff-Effizienz der Fraktionen BAC ($eC_{BAC}$) und MIC ($eC_{MIC}$) der Bodenmikroflora unter Verwendung einer Funktion vom Typ $eC = \mathbf{a}[rf/rb]^b$ erfolgt.

**11.** Verfahren zur Bewertung der Kohlenstoff-Effizienz (eC) mikrobieller Populationen des Bodens in Anwesenheit unterirdischer Pflanzenbiomassen und herbstlicher mineralischer Stickstoffrückstände (rNm) und zur Empfehlung der Verwendung von durch Inokulation wiedereingeführten Mikroorganismen, die diese Biomassen verwerten können, gemäß dem vorhergehenden Anspruch, der dadurch charakterisiert ist, dass die Koeffizienten $\underline{a}$ und $\underline{b}$ Werte von 0,54 +/- 5 bis 7 % beziehungsweise 0,65 +/- 4 bis 6 % haben.

**12.** Verfahren zur Bewertung der Kohlenstoff-Effizienz (eC) mikrobieller Populationen des Bodens in Anwesenheit unterirdischer Pflanzenbiomassen und herbstlicher mineralischer Stickstoffrückstände (rNm) und zur Empfehlung der Verwendung von durch Inokulation wiedereingeführten Mikroorganismen, die diese Biomassen verwerten können, gemäß einem der vorhergehenden Ansprüche, der dadurch charakterisiert ist, dass der Index der relativen Kohlenstoff-Effizienz, $eC_{BAC}/eC_{MIC}$, das heißt, das Verhältnis zwischen dem eC der bakteriellen (BAC) und mikrobiellen (MIC) Fraktionen des Bodens und die Aufwertung dieses Index in Bezug auf eine kritische Schwelle innerhalb von Vorhersageintervallen, deren untere Grenzen kritische Werte zwischen 0,75 und 0,92, insbesondere zwischen 0,80 und 0,90, und am besten 0,85, aufweist, was hinweist auf einen relativen Wirkungsgrad von 1,05 bis 1,1 *für eine Verwendung von durch Inokulation wiedereingeführten Mikroorganismen, die diese unterirdischen Biomassen verwerten können.*

**13.** Verfahren zur Bewertung der Kohlenstoff-Effizienz (eC) mikrobieller Populationen des Bodens in Anwesenheit unterirdischer Pflanzenbiomassen und herbstlicher mineralischer Stickstoffrückstände (rNm) und zur Empfehlung der Verwendung von durch Inokulation wiedereingeführten Mikroorganismen, die diese Biomassen verwerten können, gemäß dem vorhergehenden Anspruch, der dadurch charakterisiert ist, dass Vorhersageintervalle, deren untere Grenzen kritische Werte zwischen 0,75 und 0,92 anzeigen, festgelegt werden gemäß einer Funktion des Typs

$$Y = aX^b +/- t_n \times S \times Wurzel(1/n + (X_o - \dot{X})^2 / \Sigma X^2)$$

wobei Y der Index der relativen Kohlenstoff-Effizienz ist, a und b die Koeffizienten der Potenzfunktionen sind, die den Index mit der relativen Effizienz der (Azoto)-Bakterisation von Ernterückständen im Boden verbinden, $t_\alpha$ ist der kritische Wert von t für ein Wahrscheinlichkeitsniveau $\alpha$, n die Anzahl der Beobachtungen, $X_o$ der Beobachtungswert, X der Mittelwert der n Beobachtungen und $\Sigma X^2$ die Summe ihrer Quadrate ist.

**Claims**

1. Method for assessing the carbon efficiency (eC) of soil microbial populations in the presence of subterranean plant biomass and residual inorganic mineral nitrogen (rNm), and for the recommendation of the use of microorganisms reintroduced by inoculation that can utilize these biomasses, by transformation of agronomic and pedological data into an index of the relative carbon efficiency of the bacterial fraction (BAC) of soil microflora (MIC), $eC_{BAC}/eC_{MIC}$, **characterized in that** it comprises the following steps;

   ➤ first step, soil sampling to obtain a representative sample of the zone or soil volume in which will be incorporated the said biomasses of plant origin;

   ➤ second step, the determination of the residual mineral nitrogen (rNm) of the sample to determine as a function of rNm the N:C ratio ($rb_{MIC}$) of the soil microbial (MIC) ;

   ➤ third step, the determination of the carbon / nitrogen ratio of the soil sample ($rCN_{sol}$) in order to determine as a function of $rCN_{sol}$ the ratio between fungal (FNG) and bacterial (BAC) fractions of the soil (rFB);

   ➤ fourth step, the determination of the N:C ratio ($rb_{BAC}$) of the bacterial fraction (BAC) of the microbial flora (MIC) of the soil according to $rb_{MIC}$ once this value $rb_{MIC}$ has been adjusted according to rFB;

   ➤ fifth step, the determination of the carbon efficiency (eC) of the BAC ($eC_{BAC}$) and MIC ($eC_{MIC}$) fractions;

   ➤ sixth step, the determination of the relative carbon efficiency index, $eC_{BAC}/eC_{MIC}$, of the BAC fraction relative to that of MIC ;

   ➤ seventh step, the determination of the relative carbon efficiency index , $eC_{BAC}/eC_{MIC}$, that is, the relationship between the eC of the bacterial (BAC) and microbial (MIC) fractions of the soil , and the evaluation of this index relative to a critical threshold within a confidence interval of 0.75 and 1.00, more particularly between the values of 0.92 and 0.98, and preferably about 0.95 indicating a relative efficiency level of 1.0 compared to a non-inoculated control of reintroduced microorganisms via inoculation and utilizing such subterranean plant biomasses.

2. Method for assessing the carbon efficiency (eC) of soil microbial populations in the presence of subterranean plant biomass and residual inorganic mineral nitrogen (rNm), and for the recommendation of the use of microorganisms reintroduced by inoculation that can utilize these biomasses according to the first claim **characterized in that** the biomass of vegetable origins are cellulosic crop residues, preferably straw-like.

3. Method for assessing the carbon efficiency (eC) of soil microbial populations in the presence of subterranean plant biomass and residual inorganic mineral nitrogen (rNm), and for the recommendation of the use of microorganisms reintroduced by inoculation that can utilize these biomasses according to any one of the preceding claims, **characterized in that** the microorganisms reintroduced by inoculation that can utilize these biomasses are bacterial, and advantageously azotobacterial, i.e. able to biologically reduce diatomic nitrogen, $N_2$, in the presence of oxygen.

4. Method for assessing the carbon efficiency (eC) of soil microbial populations in the presence of subterranean plant biomass and residual inorganic mineral nitrogen (rNm), and for the recommendation of the use of microorganisms reintroduced by inoculation that can utilize these biomasses according to any one of the preceding claims, **characterized in that** the determination of $rb_{MIC}$ is a function of rNm as follows; $rb_{MIC} = a[rNm]^b$.

5. Method for assessing the carbon efficiency (eC) of soil microbial populations in the presence of subterranean plant biomass and residual inorganic mineral nitrogen (rNm), and for the recommendation of the use of microorganisms reintroduced by inoculation that can utilize these biomasses according to the preceding claim **characterized in that** the values of the coefficients a and b are 0.0068 +/- 5 to 7%, and 0.7336 +/- 4 to 6%, respectively.

6. Method for assessing the carbon efficiency (eC) of soil microbial populations in the presence of subterranean plant biomass and residual inorganic mineral nitrogen (rNm), and for the recommendation of the use of microorganisms reintroduced by inoculation that can utilize these biomasses according to any one of the preceding claims, **characterized in that** rFB is a function of $rCN_{sol is}$ as follows ; rFB (mass) = a[exp(b(rCN sol))].

7. Method for assessing the carbon efficiency (eC) of soil microbial populations in the presence of subterranean plant biomass and residual inorganic mineral nitrogen (rNm), and for the recommendation of the use of microorganisms reintroduced by inoculation that can utilize these biomasses according to the preceding claim **characterized in that** the values of the coefficients a and b are 0.008 +/- 5 to 7% and 0. 11 +/- 4 to 6%, respectively.

8. Method for assessing the carbon efficiency (eC) of soil microbial populations in the presence of subterranean plant biomass and residual inorganic mineral nitrogen (rNm), and for the recommendation of the use of microorganisms reintroduced by inoculation that can utilize these biomasses according to any one of the preceding claims, **characterized in that** the determination of $rb_{BAC}$ as function of $rb_{MIC}$ once $rb_{MIC}$ has been adjusted according to the rFB is as follows ; $rb_{BAC} = a[rb_{FNG}]^b$ given that $rb_{FNG} = (2(rb_{MIC}))/(rFB + 1)$.

9. Method for assessing the carbon efficiency (eC) of soil microbial populations in the presence of subterranean plant biomass and residual inorganic mineral nitrogen (rNm), and for the recommendation of the use of microorganisms reintroduced by inoculation that can utilize these biomasses according to the preceding claim **characterized in that** the values of the coefficients a and b are 0.52 +/- 5 to 7% and 0.47 +/- 4 to 6%, respectively.

10. Method for assessing the carbon efficiency (eC) of soil microbial populations in the presence of subterranean plant biomass and residual inorganic mineral nitrogen (rNm), and for the recommendation of the use of microorganisms reintroduced by inoculation that can utilize these biomasses according to any one of the preceding claims **characterized in that** the determination of the carbon efficiency of the BAC ($eC_{BAC}$) and MIC ($eC_{MIC}$) fractions of the soil microflora is done with a function of the type $eC = a[rf/rb]^b$.

11. Method for assessing the carbon efficiency (eC) of soil microbial populations in the presence of subterranean plant biomass and residual inorganic mineral nitrogen (rNm), and for the recommendation of the use of microorganisms reintroduced by inoculation that can utilize these biomasses according to the preceding claim **characterized in that** the values of the coefficients a and b are 0.54 +/- 5 to 7% and 0.65 +/- 4 to 6%, respectively.

12. Method for assessing the carbon efficiency (eC) of soil microbial populations in the presence of subterranean plant biomass and residual inorganic mineral nitrogen (rNm), and for the recommendation of the use of microorganisms reintroduced by inoculation that can utilize these biomasses according to any one of the preceding claims, **characterized in that** the relative carbon efficiency index, $eC_{BAC} / eC_{MIC}$, i.e. the ratio between the eC of bacterial (BAC ) and microbial (MIC) fractions of the soil, and the appreciation of this index with respect to a critical threshold within prediction intervals whose lower limits indicate critical values between 0.75 and 0.92, more particularly between 0.80 and 0.90, and preferably 0.85 indicating a relative efficiency of 1.05 to 1.1 *for microorganisms reintroduced by inoculation capable of enhancing the degradation of such underground biomasses of plant origin.*

13. Method for assessing the carbon efficiency (eC) of soil microbial populations in the presence of subterranean plant biomass and residual inorganic mineral nitrogen (rNm), and for the recommendation of the use of microorganisms reintroduced by inoculation that can utilize these biomasses according to the preceding claim **characterized in that** the prediction intervals whose lower bounds indicate critical values between 0.75 and 0.92 are established according to a function of the type,

$$Y = aX^b +/- t_n \cdot S \cdot sqrt(1 / n + ( X_o - \ddot{X})^2 / \Sigma X^2 )$$

where Y is the index relative carbon efficiency, a and b are the coefficients of the power functions relating the index to the relative effectiveness of the (azoto)bacterial treatment of the said soil borne crop residues, t is the critical value of t at probability level $\alpha$, n the number of observations, $X_o$ the value of the observation, X the mean of n-observations, and $\Sigma X^2$ the sum of their squares.

Figure 1

Figure 2

Figure 3

Figure 4

Figure 5

Figure 6

Figure 7

Figure 8

Figure 9

Figure 10

| | rb-MIC : rNm | rFB : rCN-sol | rb-BAC : rb-FNG | eC : rf/rb |
|---|---|---|---|---|
| a | 6,40 | 5,53 | 6,13 | 6,75 |
| b | 3,92 | 6,35 | 5,28 | 4,81 |

Figure 11

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- FR 0101542
- FR 0115542
- FR 0905120
- EP 2572581 A1
- FR 2995318 A1
- EP 12366001 A
- FR 1202413
- EP 2227936 A1
- EP 2409561 A1
- EP 2223586 A1

**Littérature non-brevet citée dans la description**

- **BAKKEN, LR.** Séparation and Purification of Bacteria from Soil. *Appl. Environ. Microbiol.,* 1985, vol. 49, 1482-1487
- **BARRETT ; BURKE.** Potential nitrogen immobilization in grassland soils across a soil organic matter gradient. *Soil Biol. Biochem.,* 2000, vol. 32, 1707-1716
- Effet des cultures intercalaires dans le maïs grains sur le rendement en grain, la qualité édaphique, et la teneur en azote inorganique des sols. **CLAUDE, PP.** Thèse de doctorat (Ph.D.) 1900. Université McGill, 1990, 108
- **CLAUDE, PP et al.** Effet du labour printanier et de cultures intercalaires de trèfle rouge sur le rendement du maïs grain. Can. *J. Plant Sci.,* 1993, vol. 73, 37-49
- Valorisation des résidus de culture et biofertilisation. **CLAUDE, PP.** Mémoire, Diplôme de recherche universitaire (DRU). École nationale supérieure d'agronomie, INPT, Novembre 1997
- **CLAUDE, PP ; L FILLON.** Effet de l'apport d'un inoculum bactérien aux résidus de culture de maïs-grain au sol sur le rendement et la qualité de blés d'hiver panifiables en France. *Agrosol. (depuis - Agrosolutions),* 2004, vol. 15 (1), 23-29
- **CLEVELAND, CC ; D LIPTZIN.** C:N:P stoichiometry in soil: is there a ''Redfield ratio'' for the microbial biomass?. *Biogeochemistry,* 2007, vol. 85, 235-252
- **FIERER et al.** Global patterns in belowground communities. *Ecology Letters,* 2009, vol. 12, 1-12
- **GARNIER et al.** Modelling carbon and nitrogen dynamics in a bare soit with and without strawincorporation. *European Journal of Soil Science,* Septembre 2003, vol. 54, 555-568
- **GRIFFITHS et al.** C:N:P stoichiometry and nutrient limitation of the soil microbial biomass in a grazed grassland site under experimental P limitation or excess. *Ecological Processes,* 2012, vol. 1, 6
- Microbial community and enzyme activities in prairie soil ecosystems under different management. **KATSALIROU.** M. Sc. in Soil Science. Aristotle University of Thessaloniki, 1993
- **MANZONI et al.** The global stoichiometry of litter nitrogen mineralisation. *Science,* 2008, vol. 321, 684
- **MANZONI et al.** Stoichiometric controls on carbon, nitrogen, and phosphorus dynamics in decomposing litter. *Ecological Monographs,* 2010, vol. 80 (1), 89-106
- **MARY et al.** A model for calculating nitrogen fluxes in soil using N tracing. *Soil Biol. Biochem.,* 1998, vol. 30, 1963-1979
- **MARY et al.** Interactions between décomposition of plant residues and nitrogen cycling in soil. *Plant and Soil,* 1996, vol. 181, 71-82
- **OELZE.** Respiratory protection of nitrogenase in Azotobacter species: is a widely held hypothesis unequivocally supported by experimental evidence?. *FEMS Microbiology Reviews,* 2000, vol. 24, 321-333
- **RECOUS et al.** In situ changes in gross N transformations in bare soil after addition of straw. *Soil Biol. Biochem.,* 1999, vol. 31, 119-133
- **STRICKLAND ; ROUSK.** Considering fungal:bacterial dominance in soils e Methods, controls, and ecosystem implications. *Soil Biology & Biochemistry,* 2010, vol. 42, 1385-1395
- **YEVDOKIMOV et al.** Nitrogen Immobilization by Soil Microorganisms Depending on Nitrogen Application Rates. *Eurasian Soil Science,* 2005, vol. 38 (5), 516-523
- *Translated from Pochvovedenie,* 2005, 581-589